# EUROPÄISCHE PATENTSCHRIFT

(11) **EP 0 836 594 B1**
(45) Veröffentlichungstag und Bekanntmachung des Hinweises auf die Patenterteilung: **07.03.2001**
(21) Anmeldenummer: 96922038.3
(22) Anmeldetag: 27.06.1996
(51) Int. Cl.: C07D 239/54, C07C 265/12, C07C 239/20, A01N 43/54

(54) **BENZYLHYDROXYLAMINE UND ZWISCHENPRODUKTE ZU DEREN HERSTELLUNG**
BENZYLHYDROXYLAMINES AND INTERMEDIATES USED TO PREPARE THEM
BENZYLHYDROXYLAMINES ET PRODUITS INTERMEDIAIRES UTILISES POUR LES PREPARER

(30) Priorität: 06.07.1995 DE 19524617; 26.04.1996 DE 19616719
(43) Veröffentlichungstag der Anmeldung: 22.04.1998
(73) Patentinhaber: BASF AKTIENGESELLSCHAFT, 67056 Ludwigshafen (DE)
(72) Erfinder: KLINTZ, Ralf, D-34292 Ahnatal (DE); HAMPRECHT, Gerhard, D-69469 Weinheim (DE); HEISTRACHER, Elisabeth, D-68159 Mannheim (DE); SCHÄFER, Peter, D-67308 Ottersheim (DE); ZAGAR, Cyrill, D-67061 Ludwigshafen (DE); WESTPHALEN, Karl-Otto, D-67346 Speyer (DE); WALTER, Helmut, D-67283 Obrigheim (DE); MISSLITZ, Ulf, D-67433 Neustadt (DE); MENKE, Olaf, D-67317 Altleiningen (DE); MENGES, Markus, D-68161 Mannheim (DE)
(86) Internationale Anmeldenummer: EP9602805
(87) Internationale Veröffentlichungsnummer: WO9702253

(56) Entgegenhaltungen:
- EP-A- 0 408 382
- EP-A- 0 542 685
- WO-A-93/06090
- WO-A-95/06641
- DE-A- 4 237 920

## Beschreibung

Die vorliegende Erfindung betrifft neue Benzylhydroxylamine der allgemeinen Formel I 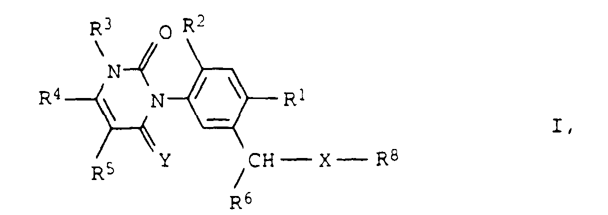 in der die Variablen folgende Bedeutung haben:
- X: -N(R⁷)-O-, das über Sauerstoff oder Stickstoff an R⁸ gebunden sein kann;
- Y: Sauerstoff oder Schwefel;
- R¹: Halogen, Cyano, Nitro oder Trifluormethyl;
- R²: Wasserstoff oder Halogen;
- R³: Wasserstoff, Amino oder Methyl;
- R⁴: Wasserstoff, Halogen, C₁-C₆-Alkyl, C₁-C₆-Halogenalkyl, C₁-C₆-Alkylthio, C₁-C₆-Alkylsulfinyl oder C₁-C₆-Alkylsulfonyl;
- R⁵: Wasserstoff, Halogen oder C₁-C₆-Alkyl;
- R⁶: Wasserstoff, C₁-C₆-Alkyl, C₁-C₆-Halogenalkyl, C₃-C₆-Cycloalkyl oder C₂-C₆-Alkenyl;
- R⁷: Wasserstoff, C₁-C₆-Alkyl, C₃-C₈-Cycloalkyl, C₁-C₆-Halogenalkyl, C₃-C₆-Alkenyl, C₃-C₆-Alkinyl, (C₁-C₆-Alkyl)carbonyl, (C₃-C₆-Alkenyl)carbonyl, (C₃-C₆-Alkinyl)carbonyl, (C₁-C₆-Alkoxy)carbonyl, (C₂-C₆-Alkenyloxy)carbonyl, (C₂-C₆-Alkinyloxy)carbonyl, (C₁-C₆-Alkylthio)carbonyl, C₁-C₆-Alkylsulfonyl, C₁-C₆-Alkylcarbamoyl, wobei jeder der letztgenannten 14 Reste gewünschtenfalls ein bis drei Substituenten tragen kann, jeweils ausgewählt aus der Gruppe bestehend aus
- Nitro, Cyano, Halogen, C₃-C₈-Cycloalkyl, Hydroxy, C₁-C₆-Alkoxy, C₃-C₈-Cycloalkoxy, C₃-C₆-Alkenyloxy, C₃-C₆-Alkinyloxy, C₁-C₆-Alkoxy-C₁-C₆-alkoxy, C₁-C₆-Alkylthio, (C₁-C₆-Alkyl)carbonyl, (C₁-C₆-Alkyl)carbonyloxy, C₁-C₆-Alkylsulfinyl, C₁-C₆-Alkylsulfonyl, C₁-C₆-Alkylidenaminoxy, C₁-C₆-Alkylcarbamoyl,
- der Phenyl-, Phenoxy- oder Phenylsulfonylgruppe, wobei die Phenylringe unsubstituiert sein oder ein bis drei Substituenten tragen können, jeweils ausgewählt aus der Gruppe bestehend aus Halogen, Nitro, Cyano, C1-C6-Alkyl, C₁-C₆-Alkoxy und C₁-C₆-Halogenalkyl,
- einer 3- bis 7-gliedrigen Heterocyclyl- oder Heterocyclyloxygruppe mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Halogen, Nitro, Cyano, C₁-C₆-Alkyl, C₁-C₆-Alkoxy, C₁-C₆-Halogenalkyl und (C₁-C₆-Alkyl)-carbonyl,
- einer Gruppe -CO-Z¹R⁹, -OCO-Z¹R⁹ oder -N(R⁹)R¹⁰,
oder
- R⁷: C₃-C₈-Cycloalkylcarbonyl, Phenylcarbonyl, Phenylsulfonyl oder Phenylcarbamoyl, wobei diese 4 Reste unsubstituiert sein oder ein bis drei Substituenten tragen können, jeweils ausgewählt aus der Gruppe bestehend aus Halogen, Nitro, Cyano, C₁-C₆-Alkyl, C₁-C₆-Alkoxy und C₁-C₆-Halogenalkyl;
- R⁸: Wasserstoff, C₁-C₆-Alkyl, C₃-C₈-cycloalkyl, C₁-C₆-Halogenalkyl, C₃-C₆-Alkenyl oder C₁-C₆-Alkinyl, wobei jeder der letztgenannten 5 Reste gewünschtenfalls ein bis drei Substituenten tragen kann, jeweils ausgewählt aus der Gruppe bestehend aus
- Nitro, Cyano, Halogen, C₃-C₈-Cycloalkyl, Hydroxy, C₁-C₆-Alkoxy, C₃-C₈-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfinyl, C₁-C₆-Alkylsulfonyl, C₁-C₆-Alkylidenaminoxy,
- der Phenyl-, Phenoxy- oder Phenylsulfonylgruppe, wobei die Phenylringe unsubstituiert sein oder ein bis drei Substituenten tragen können, jeweils ausgewählt aus der Gruppe bestehend aus Nitro, Cyano, Halogen, C₁-C₆-Alkyl, C₁-C₆-Alkoxy oder C₁-C₆-Halogenalkyl,
- einer 3- bis 7-gliedrigen Heterocyclyl- oder Heterocyclyloxygruppe mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Halogen, Nitro, Cyano, C₁-C₆-Alkyl, C₁-C₆-Alkoxy, C₁-C₆-Halogenalkyl oder (C₁-C₆-Alkyl)-carbonyl,
- einer Gruppe -CO-Z²R¹¹, -OCO-Z²R¹¹ oder -N(R¹¹)R¹²;
- Z¹: eine chemische Bindung, Sauerstoff, Schwefel oder -N(R¹⁰)-;
- Z²: eine chemische Bindung, Sauerstoff, Schwefel oder -N(R¹²)-;
- R⁹, R¹¹: unabhängig voneinander Wasserstoff, C₁-C₆-Alkyl, C₁-C₆-Halogenalkyl, C₃-C₈-Cycloalkyl, C₃-C₆-Alkenyl, C₃-C₆-Alkinyl, C₁-C₆-Alkoxy-C₁-C₆-alkyl, (C₁-C₆-Alkoxy)carbonyl-C₁-C₆-alkyl, Phenyl oder Phenyl-C₁-C₆-alkyl, wobei die Phenylgruppe und der Phenylring der Phenylalkylgruppe unsubstituiert sein oder einen bis drei Reste tragen können, jeweils ausgewählt aus der Gruppe bestehend aus Nitro, Cyano, Halogen, C₁-C₆-Alkyl, C₁-C₆-Halogenalkyl, C₁-C₆-Alkoxy oder (C₁-C₆-Alkyl)carbonyl
oder
Z¹ und R⁹ und/oder Z² und R¹¹ jeweils zusammen für einen über Stickstoff gebundenen 3- bis 7-gliedrigen Heterocyclus mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Nitro, Cyano, Halogen, C₁-C₆-Alkyl, C₁-C₆-Halogenalkyl und C₁-C₆-Alkoxy;
R¹⁰, R¹² unabhängig voneinander Wasserstoff, Hydroxy, C₁-C₆-Alkyl, C₃-C₈-Cycloalkyl oder C₁-C₆-Alkoxy,
sowie die landwirtschaftlich brauchbaren Salze derjenigen Verbindungen I, bei denen R³, R⁷ und/oder R⁸ Wasserstoff bedeuten.

Außerdem betrifft die Erfindung
- die Verwendung der Verbindungen I als Herbizide oder zur Desikkation und/oder Defoliation von Pflanzen,
- herbizide Mittel und Mittel zur Desikkation und/oder Defoliation von Pflanzen, welche die Verbindungen I als wirksame Substanzen enthalten,
- Verfahren zur Bekämpfung von unerwünschtem Pflanzenwuchs und zur Desikkation und/oder Defoliation von Pflanzen mit den Verbindungen I,
- Verfahren zur Herstellung von herbiziden Mitteln und Mitteln zur Desikkation und/oder Defoliation von Pflanzen unter Verwendung der Verbindungen I, sowie
- neue Zwischenprodukte der Formeln IV, V, XIII, XVI, XVII, XIX und XXIII aus denen die Verbindungen I erhältlich sind.

In der WO 93/06090 und der EP-A 408 382 werden bereits bestimmte 3-Phenyluracile als Herbizide und zur Desikkation/Defoliation von Pflanzen beschrieben, die sich - bei geeigneter Wahl der Substituenten - von den vorliegenden Verbindungen I insbesondere dadurch unterscheiden, daß der Phenylring anstelle des Substituenten -CH(R⁶)-X-R⁸ eine Iminomethyl-Gruppe trägt.

Gemäß der Lehre der WO 95/06641 sind u.a. Verbindungen der Formel II 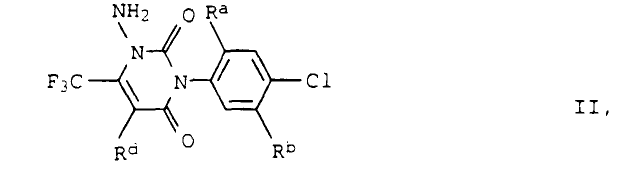 wobei R^{a} für Wasserstoff, Fluor oder Chlor und
R^{b} u.a. für eine Hydroxyiminomethyl- oder Oxyiminomethylgruppe stehen, ebenfalls als Herbizide und zur Desikkation/Defoliation von Pflanzen geeignet.

Schließlich ist aus der DE-A 42 37 920 bekannt, daß sich bestimmte 3-Aryluracile, u.a. die Verbindungen der Formel III 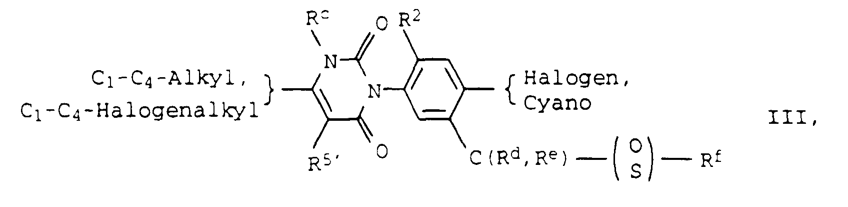 wobei
- R^{c}: für Wasserstoff, C₁-C₄-Alkyl, C₁-C₄-Haloalkyl, C₃/C₄-Alkenyl oder C₃/C₄-Alkinyl;
- R^{d} und R^{e}: für Wasserstoff, C₁-C₈-Alkyl, C₃-C₇-Cycloalkyl oder C₃-C₇-Cycloalkyl-C₁-C₄-alkyl;
- R^{f}: für Wasserstoff, C₁-C₅-Alkyl, C₁-C₄-Alkoxy-C₁-C₄-alkyl, C₃-C₈-Cycloalkyl, C₃-C₈-Cycloalkyl-C₁-C₄-alkyl, C₁-C₅-Haloalkyl, C₂-C₅-Alkenyl, C₂-C₅-Alkinyl, ggf. subst. Aryl oder Benzyl oder für eine Keto-, Ester- oder Thioester-Gruppe und
- R⁵': für Wasserstoff, Halogen oder C₁-C₄-Alkyl stehen, zur Unkrautbekämpfung eignen.

Die herbiziden Eigenschaften der bekannten Verbindungen sind jedoch nicht immer voll befriedigend. Es lagen daher dieser Erfindung neue, insbesondere herbizid wirksame Verbindungen als Aufgabe zugrunde, mit denen sich unerwünschte Pflanzen besser als bisher gezielt bekämpfen lassen.

Die Aufgabe erstreckt sich auch auf die Bereitstellung neuer desikkant/defoliant wirksamer Verbindungen.

Demgemäß wurden die Benzylhydroxylamine der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des weiteren wurde gefunden, daß die Verbindungen I auch zur Defoliation und Desikkation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und/oder Defoliation von Pflanzen, Verfahren zur Herstellung dieser Mittel und Verfahren zur Desikkation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Sofern R³, R⁷ und/oder R⁸ Wasserstoff bedeuten können die Benzylhydroxylamine I in Form ihrer landwirtschaftlich brauchbaren Salze vorliegen, wobei es auf die Art des Salzes in der Regel nicht ankommt. Im allgemeinen kommen die Salze von solchen Basen in Betracht, bei denen die herbizide Wirkung im Vergleich zu der freien Verbindung I nicht negativ beeinträchtigt ist.

Als basische Salze eignen sich besonders diejenigen der Alkalimetalle, vorzugsweise Natrium- und Kaliumsalze, der Erdalkalimetalle, vorzugsweise Calcium- und Magnesiumsalze, die der Übergangsmetalle, vorzugsweise Zink- und Eisensalze, sowie Ammoniumsalze, bei denen das Ammoniumion gewünschtenfalls ein bis vier C₁-C₄-Alkyl-, Hydroxy-C₁-C₄-alkylsubstituenten und/oder einen Phenyl- oder Benzylsubstituenten tragen kann, vorzugsweise Diisopropylammonium-, Tetramethylammonium-, Tetrabutylammonium-, Trimethylbenzylammonium- und Trimethyl-(2-hydroxyethyl)-ammoniumsalze, des weiteren Phosphoniumsalze, Sulfoniumsalze wie vorzugsweise Tri-(C₁-C₄-alkyl)sulfonium-Salze, und Sulfoxoniumsalze wie vorzugsweise Tri-(C₁-C₄-alkyl)sulfoxoniumsalze.

Die für die Substituenten R⁴ bis R¹² oder als Reste an Phenylringen- oder Heterocyclen genannten organischen Molekülteile stellen Sammelbegriffe für individuelle Aufzählungen der einzelnen Gruppenmitglieder dar. Sämtliche Kohlenstoffketten, also alle Alkyl-, Halogenalkyl-, Alkoxy-, Alkylthio-, Alkylsulfenyl-, Alkylsulfonyl-, Alkylcarbonyl-, Alkoxycarbonyl-, Alkenyl-, Alkenyloxy-, Alkenylcarbonyl-, Alkinyl-, Alkinyloxy-, Alkinylcarbonyl- und Alkylidenaminooxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise
- C₁-C₄-Alkyl sowie die Alkyl-Teile von (C₁-C₆-Alkyl)carbonyl, (C₁-C₆-Alkyl)carbonyloxy, C₁-C₆-Alkylcarbamoyl, C₁-C₆-Alkoxy-C₁-C₆-alkyl und (C₁-C₆-Alkoxy)carbonyl-C₁-C₆-alkyl: Methyl, Ethyl, n-Propyl, 1-Methylethyl, n-Butyl, 1-Methylpropyl, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, 1,1-Dimethylpropyl, 1,2-Dimethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropl, 1,2,2-Trimethylpropyl, 1-Ethyl-1-methylpropyl oder 1-Ethyl-2-methylpropyl;
- C₁-C₆-Halogenalkyl: einen C₁-C₆-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Petafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, l-(Chlormethyl)-2-chlorethyl, 1-(Brommethyl)-2-bromethyl, 4-Fluorbutyl, 4-Chlorbutyl, 4-Brombutyl, Nonafluorbutyl, 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- Phenyl-C₁-C₆-alkyl: z.B. Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 3-Phenylbut-2-yl, 4-Phenylbut-2-yl, l-(Phenylmethyl)-eth-1-yl, 1-(Phenylmethyl)-1-(methyl)-eth-1-yl oder 1- (Phenylmethyl)-prop-1-yl, vorzugsweise Benzyl, 2-Phenylethyl oder 2-Phenyl-hex-6-yl;
- C₃-C₆-Alkenyl sowie die Alkenyl-Teile von C₃-C₆-Alkenyloxy und (C₃-C₆-Alkenyl)carbonyl: Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methyl-prop-2-en-1-yl oder 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Mmethyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, l-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-put-3-en-1-yl, 1,2-Dimethyl-but-1-en-1-yl, 1,2-Dimetyl-put-2-en-1-yl, 1,2-Dimethyl-but-3-en-1-yl, 1,3-Dimetyl-put-1-en-1-yl, 1,3-Dimethyl-but-2-en-1-yl, 1,3-Dimetyl-put-3-en-1-yl, 2,2-Dimethyl-but-3-en-1-yl, 2,3-Dimetyl-put-1-en-1-yl, 2,3-Dimethyl-but-2-en-1-yl, 2,3-Dimetyl-put-3-en-1-yl, 3,3-Dimethyl-but-1-en-1-yl, 3,3-Dimetyl-put-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₃-C₆-Alkinyl sowie die Alkinyl-Teile von C₃-C₆-Alkinyloxy und (C₃-C₆-Alkinyl)carbonyl: Prop-1-in-1-yl, Prop-2-in-1-yl, But-1-in-1-yl, But-1-in-3-yl, But-1-in-4-yl, But-2-in-1-yl, Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl;
- C₂-C₆-Alkenyl sowie der Akenylteil von (C₂-C₆-Alkenyloxy)carbonyl: Vinyl oder einen der unter C₃-C₆-Alkenyl genannten Reste;
- der Alkinylteil von (C₂-C₆-Alkinyloxy)carbonyl: Ethinyl oder einen der unter C₃-C₆-Akinyl genannten Reste;
- C₁-C₆-Alkoxy sowie die Alkoxy-Teile von C₁-C₆-Alkoxy-C₁-C₆-alkyl, (C₁-C₆-Alkoxy)carbonyl und (C₁-C₆-Alkoxylcarbonyl-C₁-C₆-alkyl: Methoxy, Ethoxy, n-Propoxy, 1-Methylethoxy, 5 n-Butoxy, 1-Methylpropoxy, 2-Methylpropoxy oder 1,1-Dimethylethoxy, n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
- C₁-C₆-Alkylthio sowie der Alkylthio-Teil von (C₁-C₆-Alkylthio)carbonyl: Methylthio, Ethylthio, n-Propylthio, 1-Methylethylthio, n-Butylthio, 1-Methylpropylthio, 2-Methylpropylthio, 1,1-Dimethylethylthio, n-Pentylthio, 1-Methylbutylthio, 2-Methylbutylthio, 3-Methylbutylthio, 2,2-Di-methylpropylthio, 1-Ethylpropylthio, n-Hexylthio, 1,1-Dimethylpropylthio, 1,2-Dimethylpropylthio, 1-Methyl-pentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutyl-thio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₆-Alkylsulfinyl: Methylsulfinyl, Ethylsulfinyl, n-Propylsulfinyl, 1-Methylethylsulfinyl, n-Butylsulfinyl, 1-Methylpropylsulfinyl, 2-Methylpropylsulfinyl, 1,1-Dimethylethylsulfinyl, n-Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, n-Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, l,l,2-Trimethylproplsulfinyl, 1,2,2-Trimethylpropylsulfinyl, 1-Ethyl-1-methylpropylsulfinyl oder 1-Ethyl-2-methylpropylsulfinyl;
- C₁-C₆-Alkylsulfonyl: Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl, 1,1-Dimethylethylsulfonyl, n-Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, 1,1-Dimethylpropylsulfonyl, 1,2-Dimethylpropylsulfonyl, n-Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylproplsulfonyl, 1,2,2-Trimethylpropylsulfonyl, 1-Ethyl-1-methylpropylsulfonyl oder l-Ethyl-2-methylpropylsulfonyl;
- C₁-C₆-Alkylidenaminoxy: Acetylidenaminoxy, 1-Propylidenaminoxy, 2-Propylidenaminoxy, 1-Butylidenaminoxy, 2-Butylidenaminoxy oder 2-Hexylidenaminoxy;
- C₃-C₆-Cycloalkyl: Cyclopropyl, Cyclobutyl, Cyclopentyl oder -hexyl;
- C₃-C₈-Cycloalkyl: Cyclopropyl, Cyclobutyl, Cyclopentyl, Cyclohexyl, Cycloheptyl oder Cyclooctyl;
- C₃-C₈-Cycloalkoxy: Cyclopropyloxy, Cyclobutyloxy, Cyclopentyloxy, Cyclohexyloxy, Cycloheptyloxy oder Cyclooctyloxy.

Beispiele für 3- bis 7-gliedrige Heterocyclen sind Oxiranyl, Aziridiny, Oxetanyl, Tetrahydrofuranyl, Tetrahydrothienyl, Pyrrolidinyl, Isoxazolidinyl, Isothiazolidinyl, Pyrazolidinyl, Oxazolidinyl, Thiazolidinyl, Imidazolidinyl, Dioxolanyl wie 1,3-Dioxolan-2-yl und 1,3-Dioxolan-4-yl, Dioxanyl wie 1,3-Dioxan-2-yl und 1,3-Dioxan-4-yl, Dithianyl wie 1,3-Dithian-2-yl, des weiteren 1,2,4-Oxadiazolidinyl, 1,3,4-Oxadiazolidinyl, l,2,4-Thiadiazolidinyl, l,3,4-Thiadiazolidinyl, 1,2,4-Triazolidinyl, 1,3,4-Triazolidinyl, 2,3-Dihydrofuryl, 2,5-Dihydrofuryl, 2,3-Dihydrothienyl, 2,5-Dihydrothienyl, 2,3-Pyrrolinyl, 2,5-Pyrrolinyl, 2,3-Isoxazolinyl, 3,4-Isoxazolinyl, 4,5-Isoxazolinyl, 2,3-Isothiazolinyl, 3,4-Isothiazolinyl, 4,5-Isothiazolinyl, 2,3-Dihydropyrazolyl, 3,4-Dihydropyrazolyl, 4,5-Dihydropyrazolyl, 2,3-Dihydrooxazolyl, 3,4-Dihydrooxazolyl, Piperidinyl, Tetrahydropyridazinyl, Tetrahydropyrimidinyl, Tetrahydropyrazinyl, l,3,5-Tetrahydrotriazinyl und l,2,4-Tetrahydrotriazinyl,
sowie die folgenden Heteroaromaten:
Furyl wie 2-Furyl und 3-Furyl, Thienyl wie 2-Thienyl und 3-Thienyl, Pyrrolyl wie 2-Pyrrolyl und 3-Pyrrolyl, Isoxazolyl wie 3-Isoxazolyl, 4-Isoxazolyl und 5-Isoxazolyl, Isothiazolyl wie 3-Isothiazolyl, 4-Isothiazolyl und 5-Isothiazolyl, Pyrazolyl wie 3-Pyrazolyl, 4-Pyrazolyl und 5-Pyrazolyl, Oxazolyl wie 2-Oxazolyl, 4-Oxazolyl und 5-Oxazolyl, Thiazolyl wie 2-Thiazolyl, 4-Thiazolyl und 5-Thiazolyl, Imidazolyl wie 2-Imidazolyl und 4-Imidazolyl, Oxadiazolyl wie 1,2,4-Oxadiazol-3-yl, 1,2,4-Oxadiazol-5-yl und 1,3,4-Oxadiazol-2-yl, Thiadiazolyl wie 1,2,4-Thiadiazol-3-yl, 1,2,4-Thiadiazol-5-yl und 1,3,4-Thiadiazol-2-yl, Triazolyl wie 1,2,4-Triazol-1-yl, 1,2,4-Triazol-3-yl und 1,2,4-Triazol-4-yl, Pyridinyl wie 2-Pyridinyl, 3-Pyridinyl und 4-Pyridinyl, Pyridazinyl wie 3-Pyridazinyl und 4-Pyridazinyl, Pyrimidinyl wie 2-Pyrimidinyl, 4-Pyrimidinyl und 5-Pyrimidinyl, des weiteren 2-Pyrazinyl, 1,3,5-Triazin-2-yl und 1,2,4-Triazin-3-yl, insbesondere Pyridyl, Pyrimidyl, Furanyl und Thienyl.

Alle Phenyl- und heterocyclischen Ringe sind vorzugsweise unsubstituiert oder tragen einen Halogen-, Methyl-, Trifluormethyl- oder Methoxy-Substituenten.

Im Hinblick auf die Verwendung der erfindungsgemäßen Verbindungen der Formel I als Herbizide und/oder als defoliant/desikkant wirksame Verbindungen haben die Variablen vorzugsweise folgende Bedeutungen, und war jeweils für sich allein oder in Kombination:
- X: über Sauerstoff an R⁸ gebundenes -N(R⁷)-O-;
- X: über Stickstoff an R⁸ gebundenes -O-N(R⁷)-;
- Y: Sauerstoff;
- R¹: Halogen oder Cyano;
- R²: Wasserstoff, Fluor oder Chlor;
- R³: Amino oder Methyl;
- R⁴: C₁-C₆-Alkyl, C₁-C₆-Halogenalkyl oder C₁-C₆-Alkylsulfonyl, besonders bevorzugt C₁-C₄-Halogenalkyl, insbesondere Trifluormethyl, Chlordifluormethyl oder Pentafluorethyl;
- R⁵: Wasserstoff oder Halogen;
- R⁶: Wasserstoff oder C₁-C₄-Alkyl, besonders bevorzugt Wasserstoff;
- R⁷: Wasserstoff, C₁-C₆-Alkyl, C₃-C₈-Cycloalkyl, C₁-C₆-Halogenalkyl, C₃-C₆-Alkenyl, C₃-C₆-Alkinyl, (C₁-C₆-Alkyl)carbonyl, (C₃-C₆-Alkenyl)carbonyl, (C₃-C₆-Alkinyl)carbonyl, (C₁-C₆-Alkoxy)carbonyl, (C₂-C₆-Alkenyloxy)carbonyl, (C₂-C₆-Alkinyloxy)carbonyl, (C₁-C₆-Alkylthio)carbonyl, C₁-C₆-Alkylcarbamoyl, wobei jeder der letztgenannten 13 Reste gewünschtenfalls einen oder zwei Substituenten tragen kann, jeweils ausgewählt aus der Gruppe bestehend aus
- Nitro, Cyano, Halogen, C₃-C₈-Cycloalkyl, Hydroxy, C₁-C₆-Alkoxy, C₃-C₈-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfenyl, C₁-C₆-Alkylsulfonyl, C₁-C₆-Alkylidenaminoxy,
- einer Gruppe -CO-Z¹R⁹, -OCO-Z¹R⁹ oder -N(R⁹)R¹⁰;
- R⁸: Wasserstoff, C₁-C₆-Alkyl, C₃-C₈-Cycloalkyl, C₁-C₆-Halogenalkyl, C₃-C₆-Alkenyl oder C₃-C₆-Alkinyl, wobei jeder der letztgenannten 5 Reste gewünschtenfalls einen oder zwei Substituenten tragen kann, jeweils ausgewählt aus der Gruppe bestehend aus
- Nitro, Cyano, Halogen, C₃-C₈-Cycloalkyl, Hydroxy, C₁-C₆-Alkoxy, C₃-C₈-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfenyl, C₁-C₆-Alkylsulfonyl, C₁-C₆-Alkylidenaminoxy,
- einer Gruppe -CO-Z²R¹¹, -OCO-Z²R¹¹ oder -N(R¹¹)R¹²;
- Z¹: eine chemische Bindung, Sauerstoff, Schwefel oder -N(R¹⁰)-;
- Z²: eine chemische Bindung, Sauerstoff, Schwefel oder -N(R¹²)-;
- R⁹, R¹¹: unabhängig voneinander Wasserstoff, C₁-C₆-Alkyl, C₃-C₈-Cycloalkyl, C₃-C₆-Alkenyl, C₃-C₆-Alkinyl, C₁-C₆-Alkoxy-C₁-C₆-alkyl oder (C₁-C₆-Alkoxy)carbonyl-C₁-C₆-alkyl;
oder
- Z¹ und R⁹: und/oder Z² und R¹¹ jeweils zusammen für einen über Stickstoff gebundenen 3- bis 7-gliedrigen Heterocyclus mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Nitro, Cyano, Halogen, C₁-C₆-Alkyl, C₁-C₆-Halogenalkyl und C₁-C₆-Alkoxy;
- R¹⁰, R¹²: unabhängig voneinander Wasserstoff oder C₁-C₆-Alkyl.

R⁷ steht besonders bevorzugt für:
Wasserstoff, C₁-C₆-Alkyl, C₃-C₈-Cycloalkyl, C₁-C₆-Haloalkyl, C₃-C₆-Alkenyl, C₃-C₆-Alkinyl, (C₁-C₆-Alkyl)carbonyl, (C₃-C₆-Alkenyl)carbonyl, (C₃-C₆-Alkinyl)carbonyl, (C₁-C₆-Alkoxy)carbonyl oder C₁-C₆-Alkylcarbamoyl, wobei jeder der letztgenannten 10 Reste noch einen der folgenden Substituenten tragen kann: Nitro, Cyano, Halogen, C₃-C₈-Cycloalkyl, Hydroxy, C₁-C₆-Alkoxy, C₃-C₈-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfenyl, C₁-C₆-Alkylsulfonyl, C₁-C₆-Alkylidenaminoxy, eine Gruppe -CO-Z¹R⁹, -OCO-Z¹R⁹ oder -N(R⁹)R¹⁰.

R⁸ steht besonders bevorzugt für:
Wasserstoff, C₁-C₆-Alkyl, C₃-C₈-Cycloalkyl, C₁-C₆-Halogenalkyl, C₃-C₆-Alkenyl oder C₃-C₆-Alkinyl, wobei jeder der letztgenannten 5 Reste noch einen der folgenden Substituenten tragen kann: Nitro, Cyano, Halogen, C₃-C₈-Cycloalkyl, Hydroxy, C₁-C₆-Alkoxy, C₃-C₈-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfinyl, C₁-C₆-Alkylsulfonyl, C₁-C₆-Alkylidenaminoxy, eine Gruppe -CO-Z²R¹¹, -OCO-Z²R¹¹ oder -N(R¹¹)R¹².

Ganz besonders bevorzugt sind die in der folgenden Tabelle 1 aufgeführten Verbindungen Ia (≙ I mit X = -NH-O-, Y = Sauerstoff, R¹ = Chlor, R² = Fluor, R³ = Amino, R⁴ = Trifluormethyl und R⁵, R⁶ = Wasserstoff):

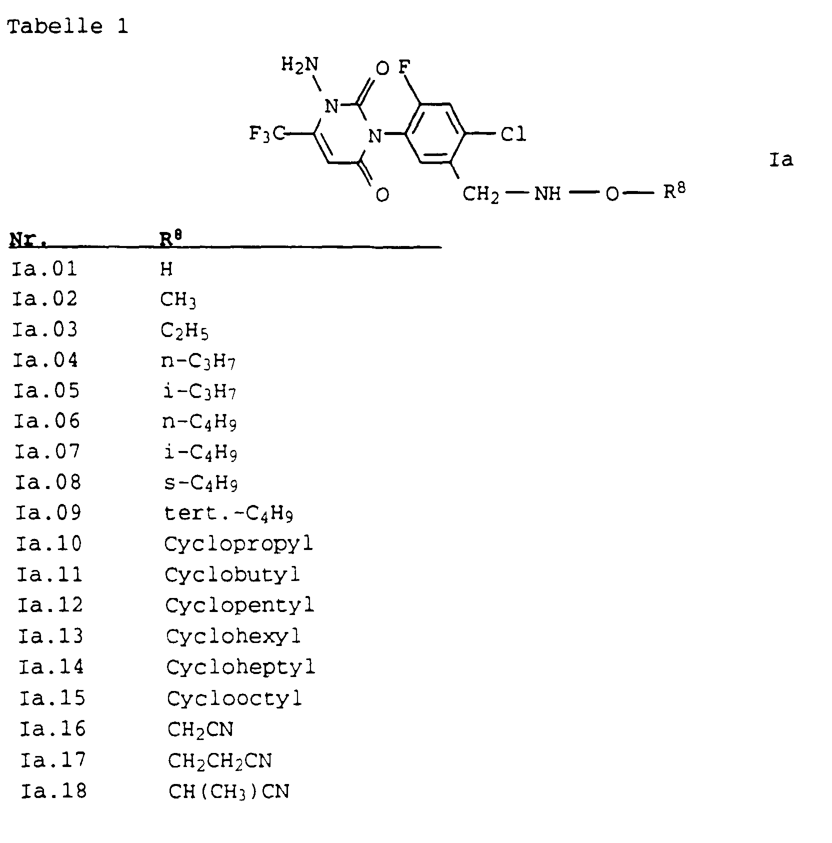

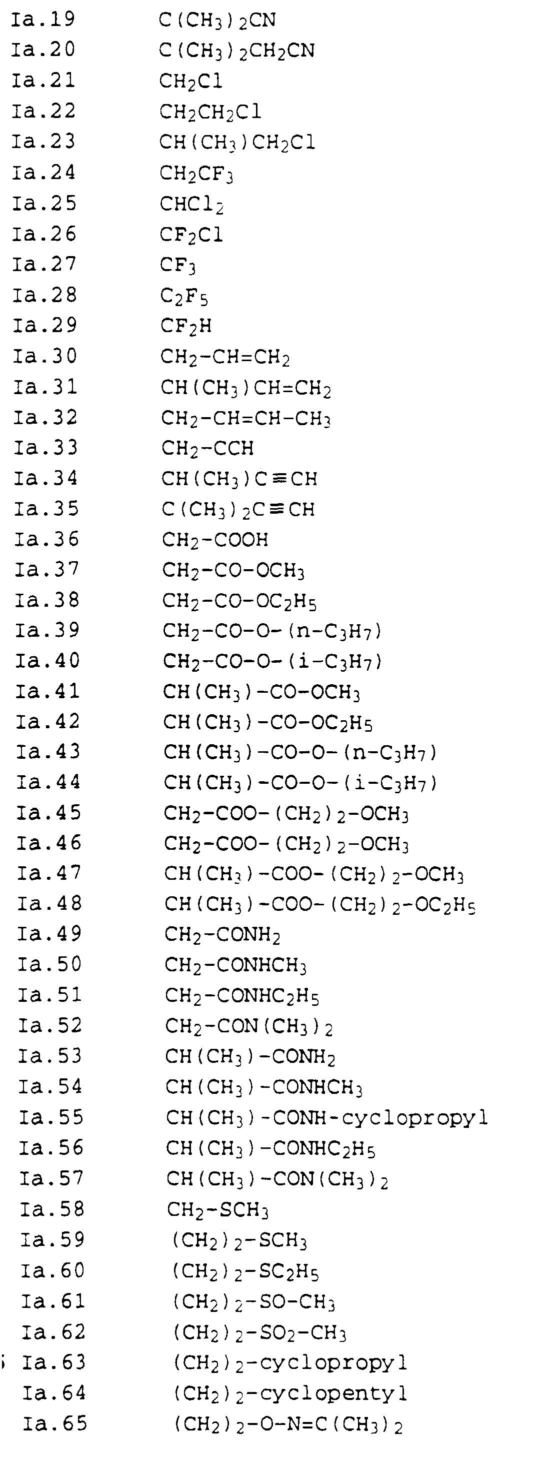

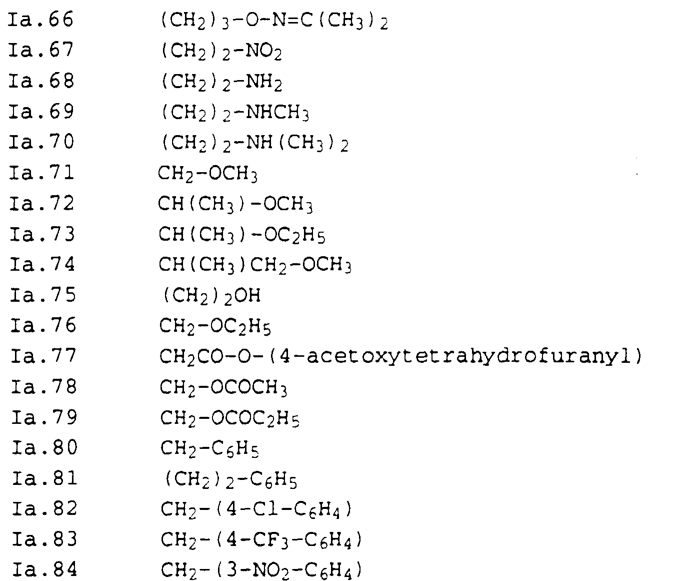

Des weiteren sind die folgenden Benzylhydroxylamine der Formel I ganz besonders bevorzugt:
- die Verbindungen Ib.01 - Ib.84, die sich von den entsprechenden Verbindungen Ia.01 - Ia.84 lediglich dadurch unterscheiden, daß R¹ für Cyano steht: 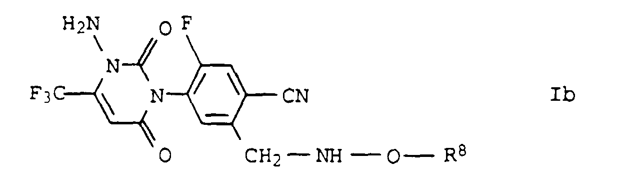
- die Verbindungen Ic.01 - Ic.84, die sich von den entsprechenden Verbindungen Ia.01 - Ia.84 lediglich dadurch unterscheiden, daß R² für Wasserstoff steht: 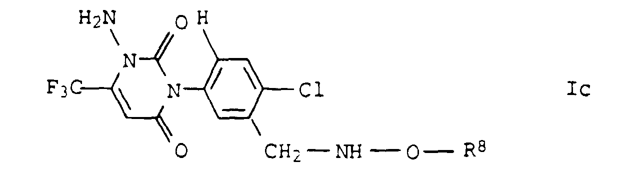
- die Verbindungen Id.01 - Id.84, die sich von den entsprechenden Verbindungen Ia.01 - Ia.84 lediglich dadurch unterscheiden, daß R¹ für Cyano und R² für Wasserstoff stehen: 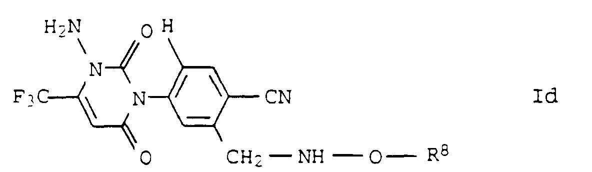
- die Verbindungen Ie.01 - Ie.84, die sich von den entsprechenden Verbindungen Ia.01 - Ia.84 lediglich dadurch unterscheiden, daß R³ für Methyl steht: 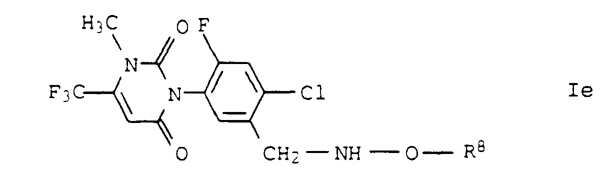
- die Verbindungen If.01 - If.84, die sich von den entsprechenden Verbindungen Ia.01 - Ia.84 lediglich dadurch unterscheiden, daß R¹ für Cyano und R³ für Methyl stehen: 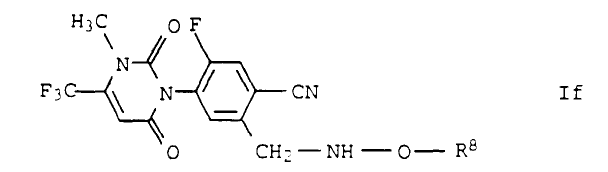
- die Verbindungen Ig.01 - Ig.84, die sich von den entsprechenden Verbindungen Ia.01 - Ia.84 lediglich dadurch unterscheiden, daß R² für Wasserstoff und R³ für Methyl stehen: 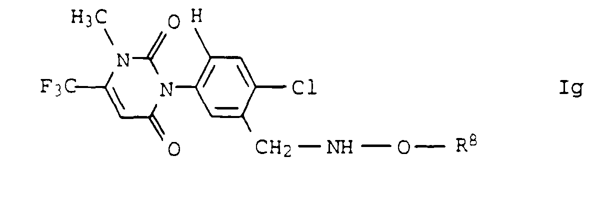
- die Verbindungen Ih.01 - Ih.84, die sich von den entsprechenden Verbindungen Ia.01 - Ia.84 lediglich dadurch unterscheiden, daß R¹ für Cyano, R² für Wasserstoff und R³ für Methyl stehen:

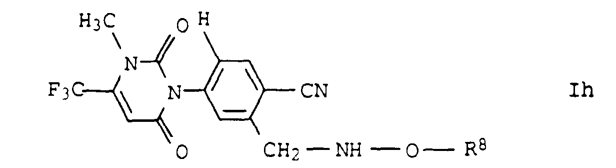

Weiterhin sind die in der folgenden Tabelle 2 aufgeführten Verbindungen Ii (≙ I mit Y = Sauerstoff, R¹ = Chlor, R² = Fluor, R³ = Amino, R⁴ = Trifluormethyl, R⁵ und R⁶ = Wasserstoff, R⁸ = Methyl) ganz besonders bevorzugt:

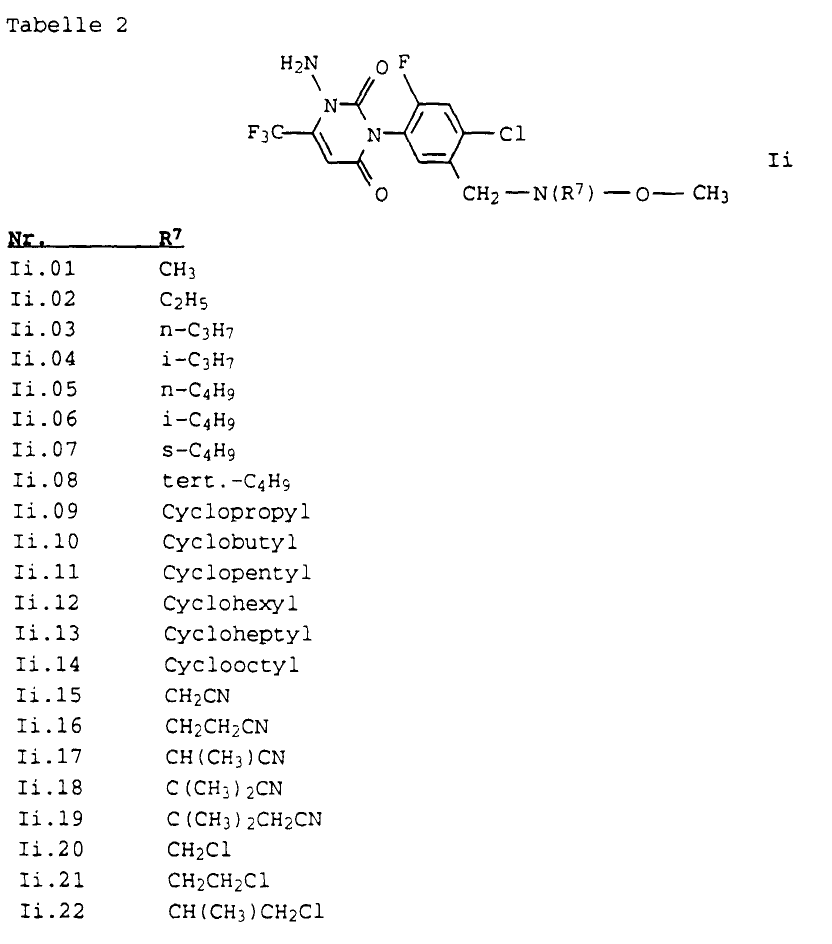

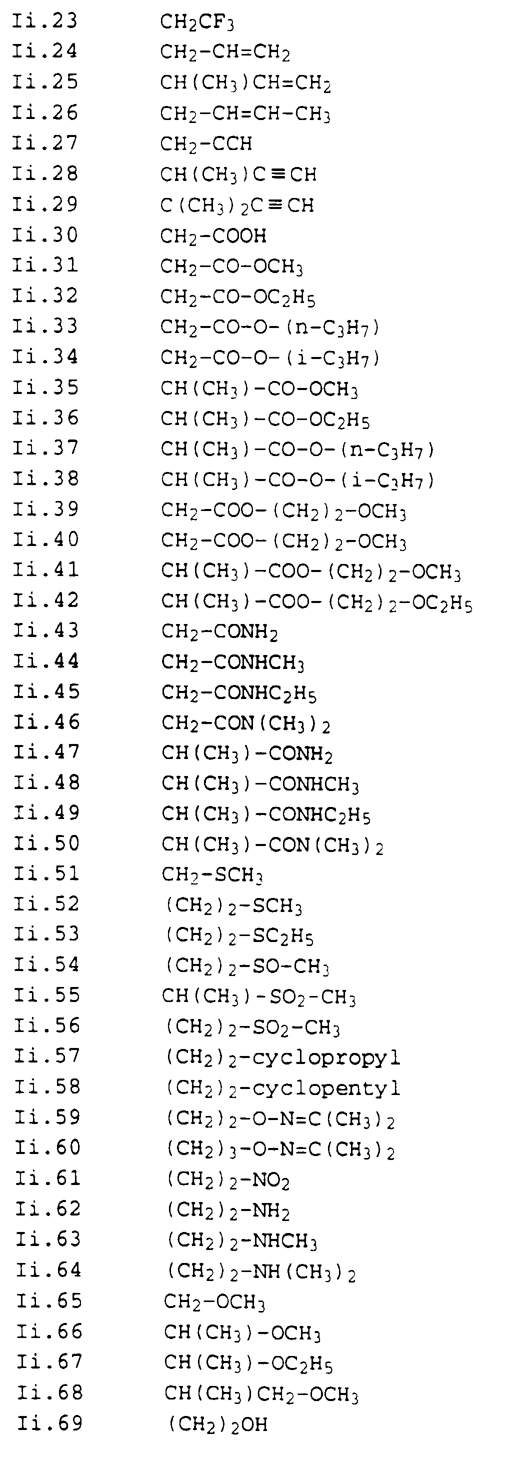

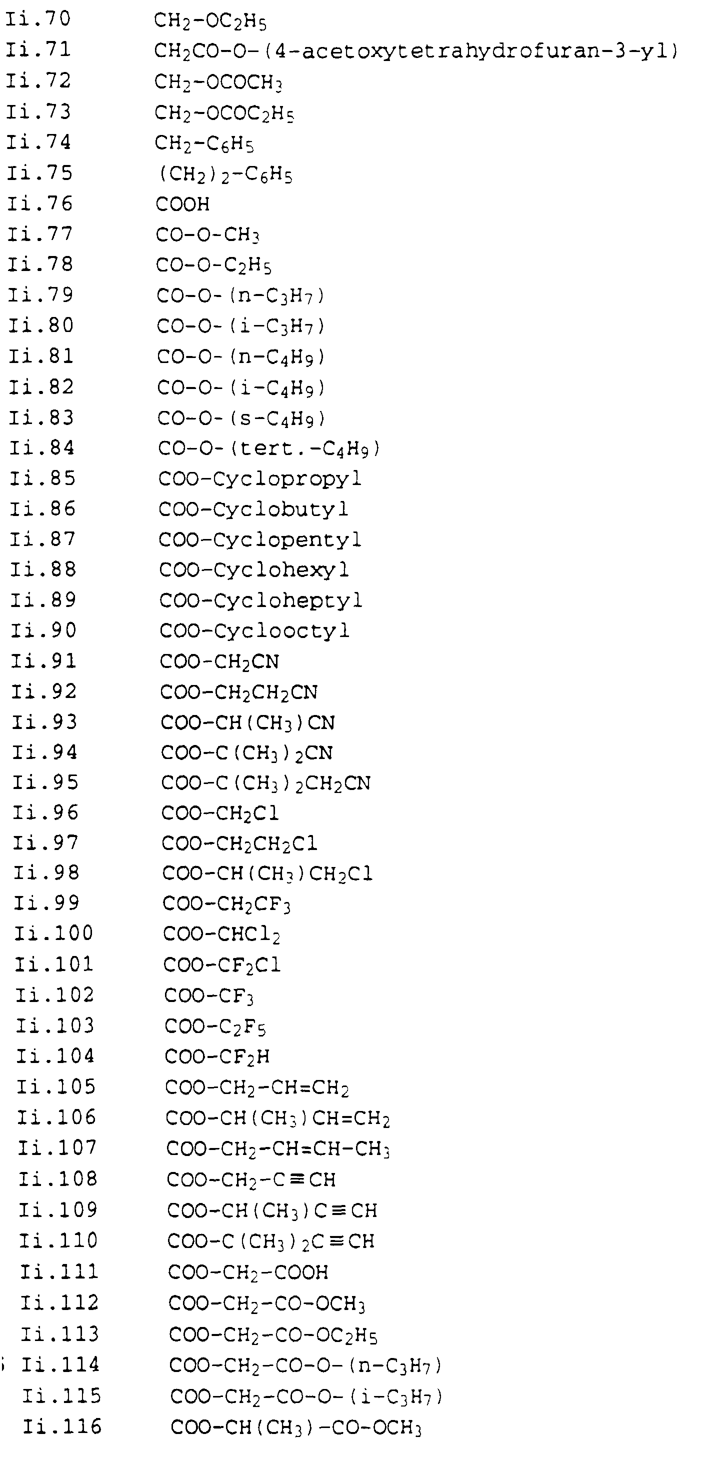

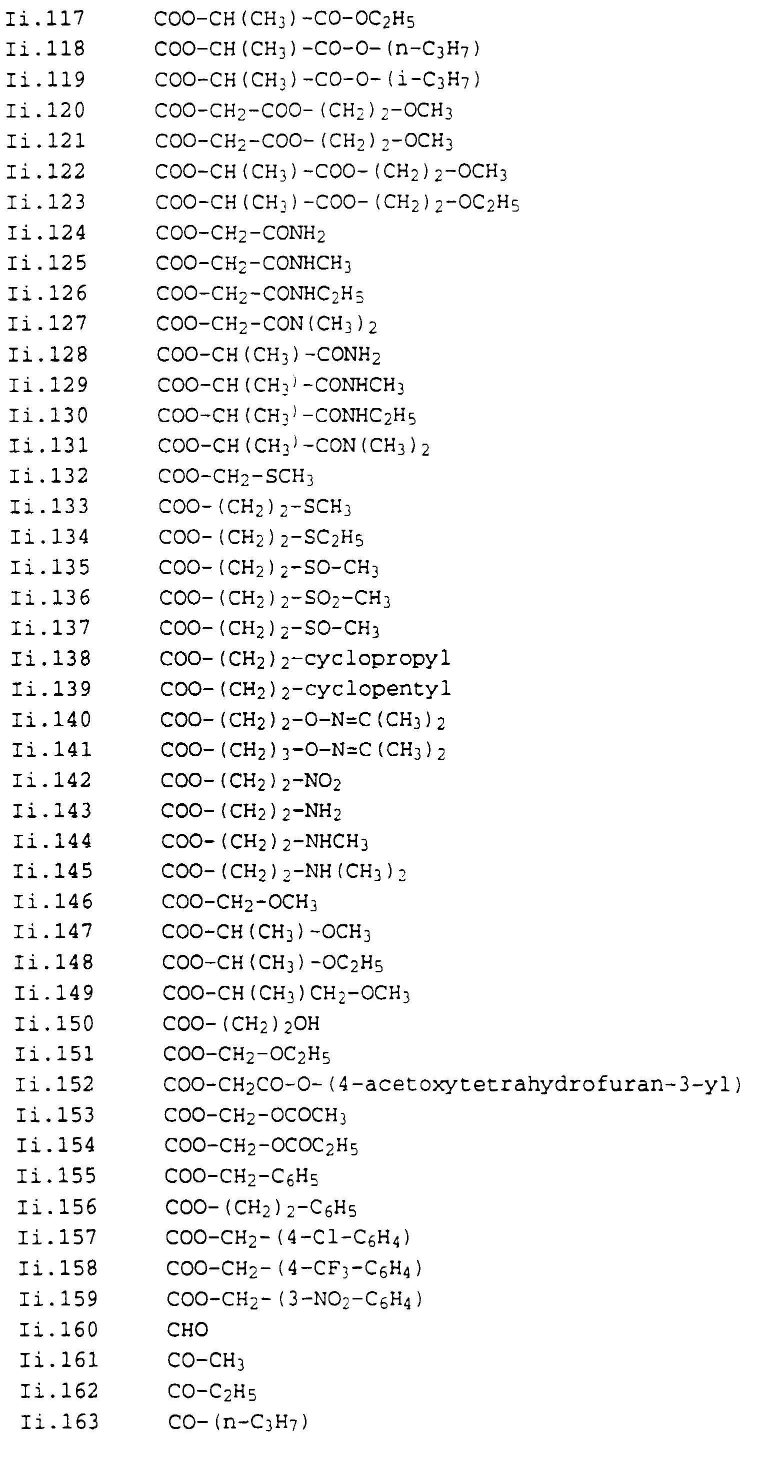

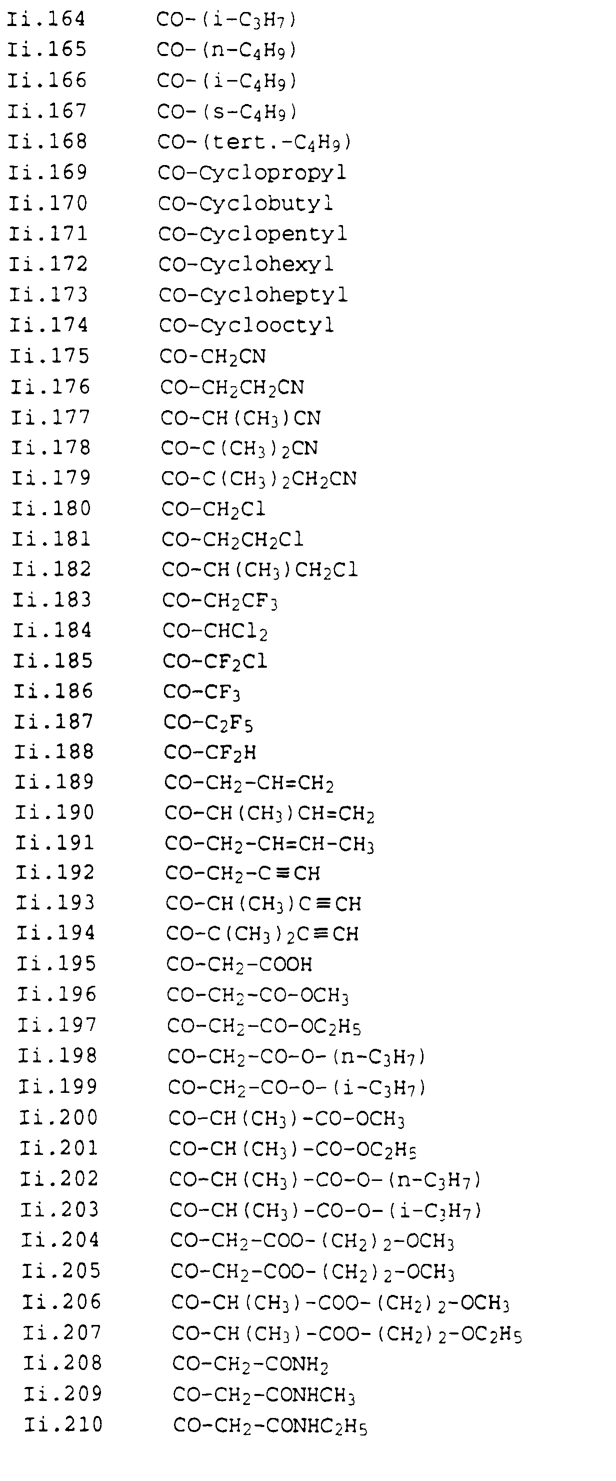

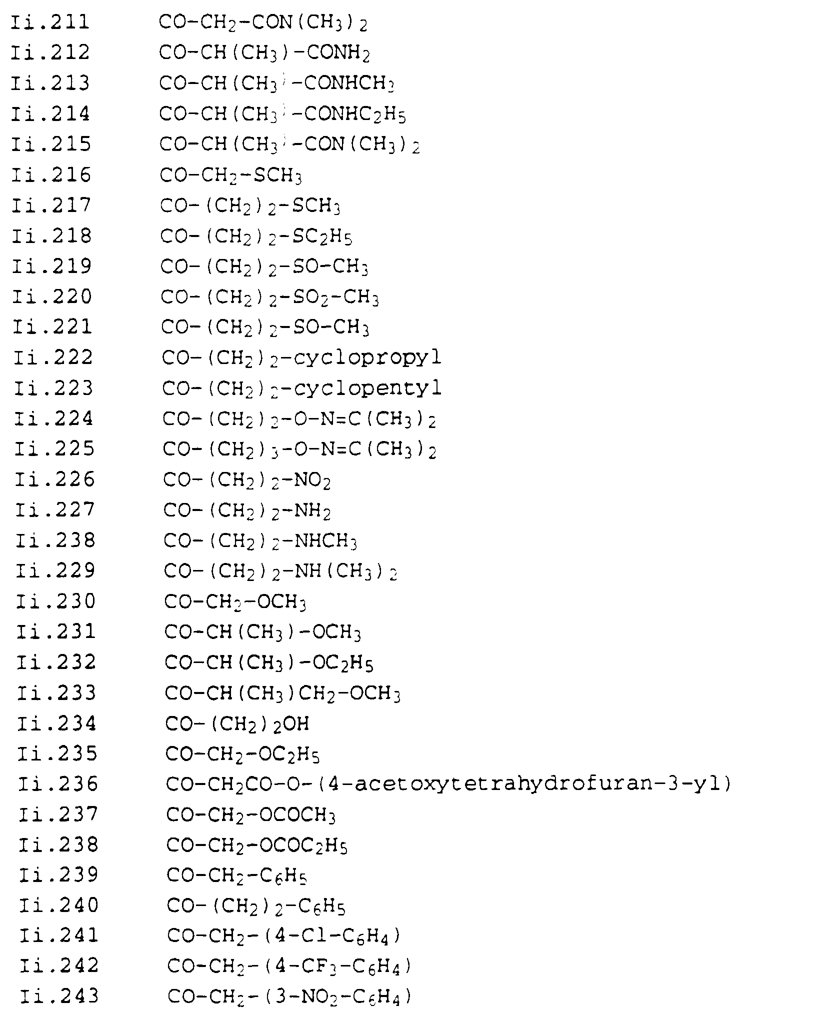

Außerdem sind die folgenden Benzylhydroxylamine I ganz besonders bevorzugt:
- die Verbindungen Ik.98 - Ik.243, die sich von den entsprechenden Verbindungen Ii.01 - Ii.243 lediglich dadurch unterscheiden, daß R¹ für Cyano steht: 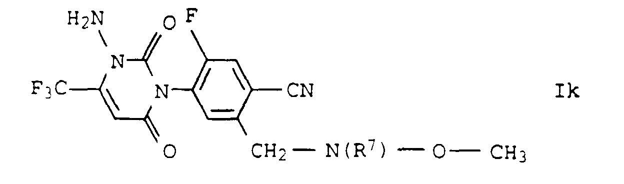
- die Verbindungen I1.01 - I1.243, die sich von den entsprechenden Verbindungen Ii.01 - Ii.243 lediglich dadurch unterscheiden, daß R² für Wasserstoff steht: 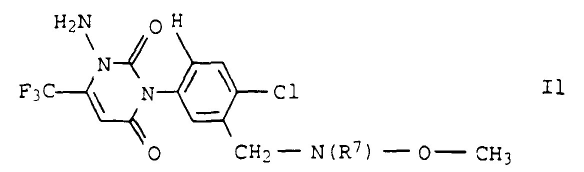
- die Verbindungen Im.01 - Im.243, die sich von den entsprechenden Verbindungen Ii.01 - Ii.243 lediglich dadurch unterscheiden, daß R¹ für Cyano und R² für Wasserstoff stehen: 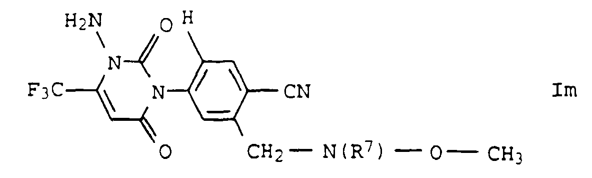
- die Verbindungen In.01 - In.243, die sich von den entsprechenden Verbindungen Ii.01 - Ii.243 lediglich dadurch unterscheiden, daß R³ für Methyl steht: 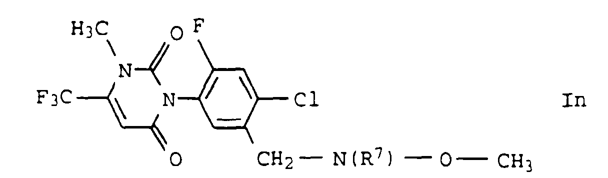
- die Verbindungen Io.01 - Io.243, die sich von den entsprechenden Verbindungen Ii.01 - Ii.243 lediglich dadurch unterscheiden, daß R¹ für Cyano und R³ für Methyl stehen: 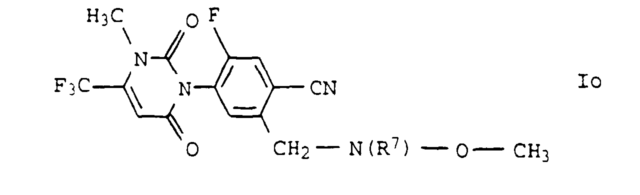
- die Verbindungen Ip.01 - Ip.243, die sich von den entsprechenden Verbindungen Ii.01 - Ii.243 lediglich dadurch unterscheiden, daß R² für Wasserstoff und R³ für Methyl stehen: 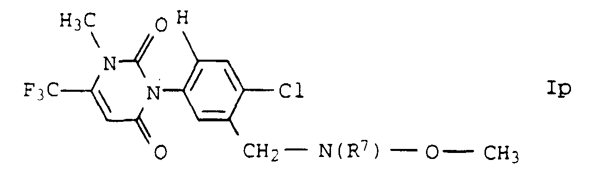
- die Verbindungen Iq.01 - Iq.243, die sich von den entsprechenden Verbindungen Ii.01 - Ii.243 lediglich dadurch unterscheiden, daß R¹ für Cyano, R² für Wasserstoff und R³ für Methyl stehen:

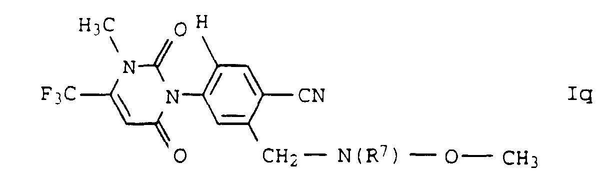

Zusätzlich sind die in der folgenden Tabelle 3 aufgeführten Verbindungen Ir (≙ I mit Y = Sauerstoff, R¹ = Chlor, R³ = Methyl, R⁴ = Trifluormethyl, R⁵ und R⁶ = Wasserstoff) ganz besonders bevorzugt:

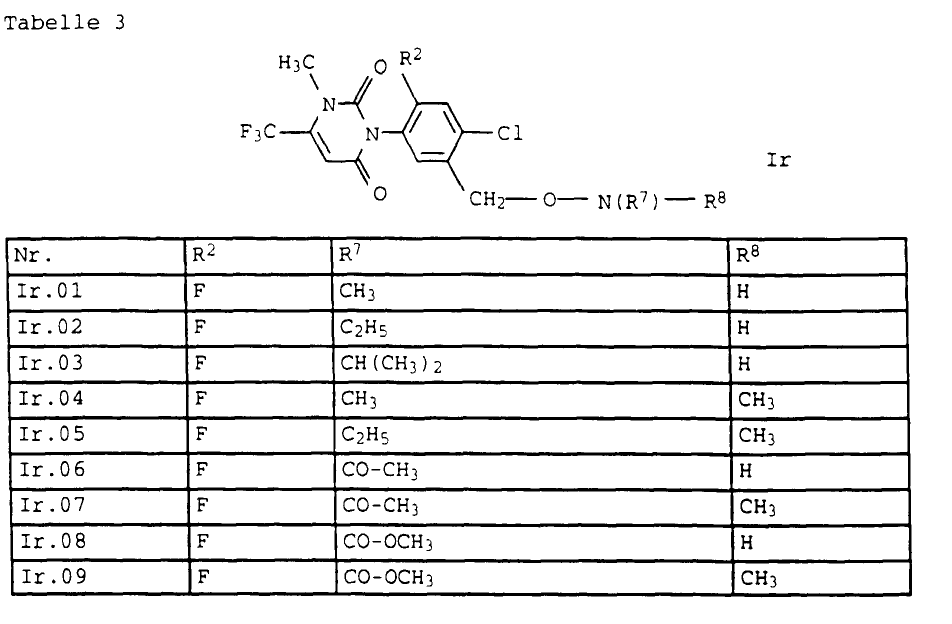

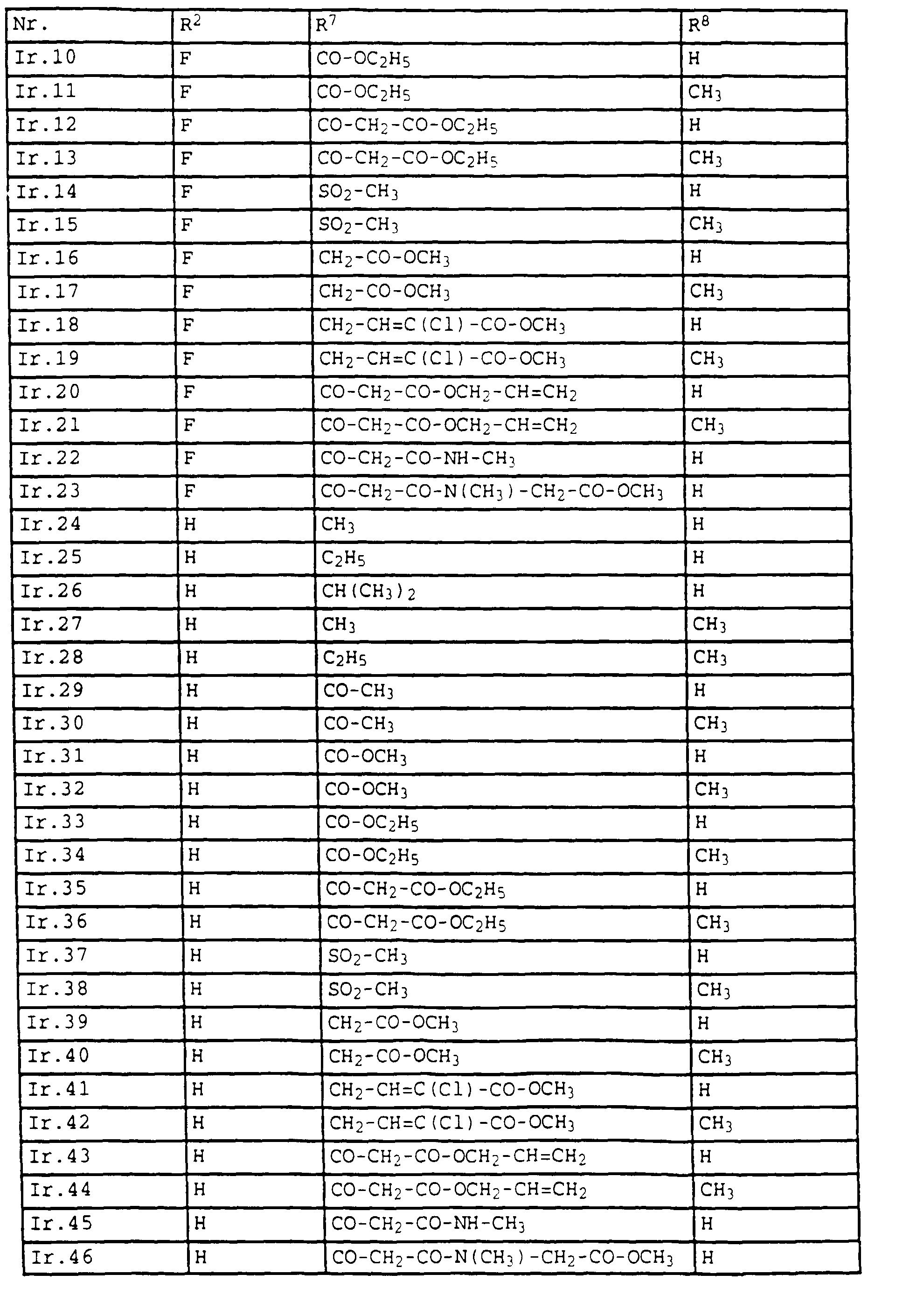

Schließlich sind noch die folgenden Benzylhydroxylamine I ganz besonders bevorzugt:
- die Verbindungen Is.01 - Is.46, die sich von den entsprechenden Verbindungen Ir.01 - Ir.46 lediglich dadurch unterscheiden, daß R¹ für Cyano steht: 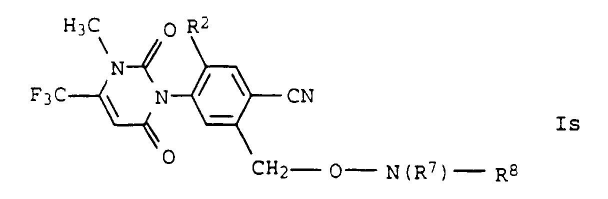
- die Verbindungen It.01 - It.46, die sich von den entsprechenden Verbindungen Ir.01 - Ir.46 lediglich dadurch unterscheiden, daß R³ für Amino steht: 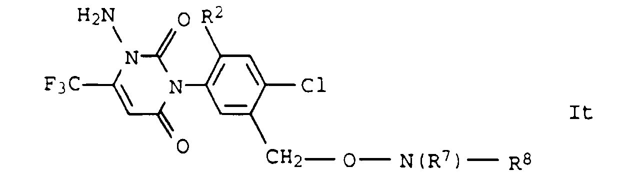
- die Verbindungen Iu.01 - Iu.46, die sich von den entsprechenden Verbindungen Ir.01 - Ir.46 lediglich dadurch unterscheiden, daß R¹ für Cyano und R³ für Amino stehen:

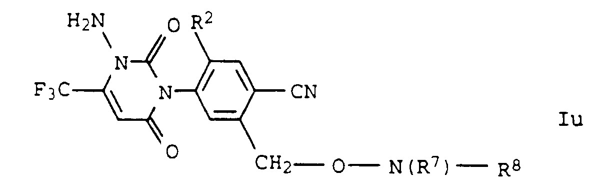

Die Benzylhydroxylamine der Formel I sind auf verschiedene Weise erhältlich, beispielsweise nach einem der folgenden Verfahren:

### Verfahren A)

cyclisierung eines Enaminesters der Formel IV oder eines Enamincarboxylats der Formel V in Gegenwart einer Base:

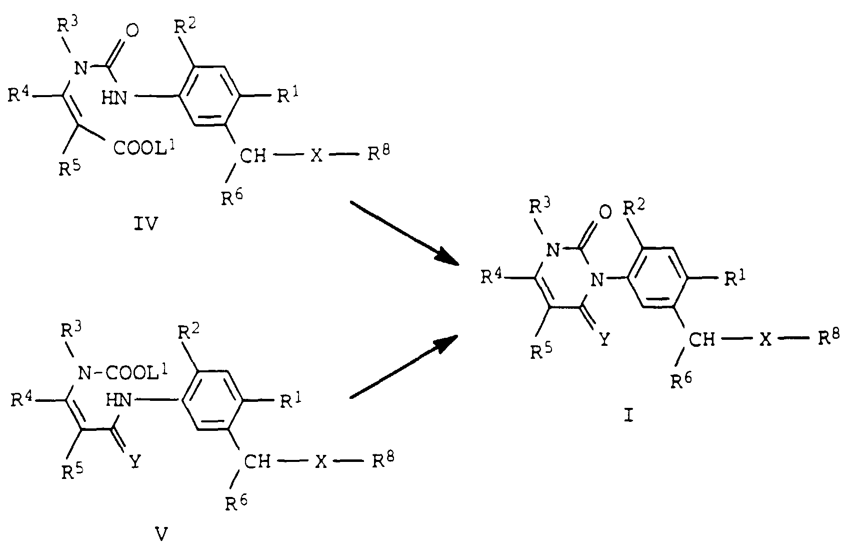

L¹ bedeutet niedermolekulares Alkyl, vorzugsweise C₁-C₄-Alkyl, oder Phenyl.

In der Regel cyclisiert man in einem inerten organischen Lösungs- oder Verdünnungsmittel, das aprotisch ist, beispielsweise in einem aliphatischen oder cyclischen Ether wie 1,2-Dimethoxyethan, Tetrahydrofuran und Dioxan, in einem Aromaten wie Benzol und Toluol oder in einem polaren Solvens wie Dimethylformamid und Dimethylsulfoxid. Auch Mischungen aus polarem Solvens und einem Kohlenwasserstoff wie n-Hexan sind geeignet. Je nach Ausgangsverbindung kann auch Wasser als Verdünnungsmittel geeignet sein.

Als Basen kommen vorzugsweise Alkalimetallalkoholate, insbesondere die Natriumalkoholate, Alkalimetallhydroxide, insbesondere Natriumhydroxid und Kaliumhydroxid, Alkalimetallcarbonate, insbesondere Natriumcarbonat und Kaliumcarbonat, und Metallhydride, insbesondere Natriumhydrid, in Betracht. Bei der Verwendung von Natriumhydrid als Base hat es sich als vorteilhaft erwiesen, in einem aliphatischen oder cyclischen Äther, in Dimethylformamid oder in Dimethylsulfoxid zu arbeiten.

Normalerweise ist die 0,5- bis zweifache molare Menge an Base, bezogen auf die Menge an IV oder V, für das Gelingen der Reaktion ausreichend.

Im allgemeinen liegt die Reaktionstemperatur bei (-78)°C bis zur Höhe der Siedetemperatur des jeweiligen Reaktionsgemisches, insbesondere bei (-60) bis 60°C.

Bedeutet R³ in Formel IV oder V Wasserstoff, so wird das Verfahrensprodukt als Metallsalz erhalten, wobei das Metall dem Kation der verwendeten Base entspricht. Das Salz kann auf an sich bekannte Weise isoliert und gereinigt oder gewünschtenfalls mittels Säure in die freie Verbindung I mit R³ = Wasserstoff übergeführt werden.

### Verfahren B)

Methylierung einer Verbindung I, bei der R³ Wasserstoff bedeutet, in Gegenwart einer Base:

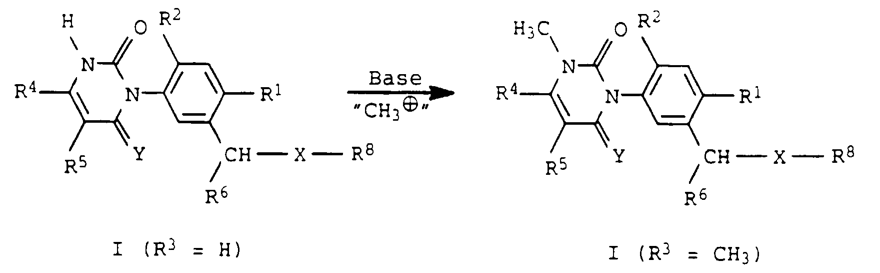

Als Methylierungsmittel kommen beispielsweise Methylhalogenide, vorzugsweise Methylchlorid, -iodid oder -bromid, sowie Dimethylsulfat, Methansulfonat (Methylmesylat), Methylbenzolsulfonat, Methan-p-tolylsulfonat (Methyltosylat), Methan-p-brombenzolsulfonat (Methylbrosylat), Trifluormethansulfonylmethan (Methyltriflat) und Diazomethan in Betracht.

In der Regel arbeitet man in einem inerten organischen Lösungsmittel, beispielsweise in einem protischen Lösungsmittel wie den niederen Alkoholen, vorzugsweise in Ethanol, gegebenenfalls im Gemisch mit Wasser, oder in einem aprotischen Lösungsmittels, z.B. in einem aliphatischen oder cyclischen Äther, vorzugsweise in 1,2-Dimethoxyethan, Tetrahydrofuran oder Dioxan, in einem aliphatischen Keton, vorzugsweise in Aceton, in einem Amid, vorzugsweise in Dimethylformamid, in einem Sulfoxid, vorzugsweise in Dimethylsulfoxid, in einem Harnstoff wie Tetramethylharnstoff und 1,3-Dimethyltetrahydro2(1H)-pyrimidinon, in einem Carbonsäureester wie Essigsaurethylester, oder in einem halogenierten aliphatischen oder aromatischen Kohlenwasserstoff wie Dichlormethan und Chlorbenzol.

Als Base eignen sich anorganische Basen, z.B. Carbonate wie Natriumcarbonat und Kaliumcarbonat, Hydrogencarbonate wie Natrium- und Kaliumhydrogencarbonat, oder Alkalimetallhydride wie Natriumhydrid und Kaliumhydrid, sowie organische Basen, z.B. Amine wie Triethylamin, Pyridin und N,N-Diethylanilin, oder Alkalimetallalkoholate wie Natriummethanolat, Natriumethanolat und Kalium-tert.-butanolat.

Die Menge an Base und Methylierungsmittel liegt vorzugsweise jeweils bei der 0,5- bis zweifachen molaren Menge, bezogen auf die Menge an Ausgangsverbindung.

Im allgemeinen liegt die Reaktionstemperatur bei 0°C bis zur Höhe der Siedetemperatur des Reaktionsgemisches, insbesondere bei 0 bis 60°C.

Eine bevorzugte Verfahrensvariante besteht darin, das aus der Cyclisierung von IV (R³ = H) oder V (R³ = H) gemäß Verfahren A) erhaltene Salz von I ohne Isolierung aus der Reaktionsmischung, die noch überschüssige Base, z.B. Natriumhydrid, Natriumalkoholat oder Natriumcarbonat, enthalten kann, zu methylieren.

Sofern nicht unmittelbar durch die als Methode A) beschriebene Cyclisierung unter basischen Bedingungen herstellbar, können die Salze derjenigen Verbindungen I, in denen R³ Wasserstoff bedeutet, auch in an sich bekannter Weise aus den Verfahrensprodukten der Methode A) erhalten werden. Zu diesem Zweck versetzt man beispielsweise die wäßrige Lösung einer anorganischen oder organischen Base mit dem Benzylhydroxylamin I, bei dem R³ für Wasserstoff steht. Die Salzbildung erfolgt normalerweise bereits bei 20-25°C mit ausreichender Geschwindigkeit.

Besonders vorteilhaft ist es, das Natriumsalz durch Auflösen des Benzylhydroxylamins I (R³ = Wasserstoff) in wäßriger Natriumhydroxidlösung bei 20-25°C herzustellen, wobei in etwa äquivalente Mengen an Benzylhydroxylamin und Natriumhydroxid eingesetzt werden. Das Salz des Benzylhydroxylamins kann dann z.B. durch Fällen mit einem geeigneten inerten Lösungsmittel oder durch Abdampfen des Lösungsmittels isoliert werden.

Salze der Benzylhydroxylamine I (R³ = H), deren Metallion kein Alkalimetallion ist, können üblicherweise durch Umsalzen des entsprechenden Alkalimetallsalzes in wäßriger Lösung hergestellt werden. Auf diese Weise lassen sich z.B. Benzylhydroxylamin-Metallsalze herstellen, die in Wasser unlöslich sind.

### Verfahren C)

Umsetzung eines Benzylhydroxylamins der Formel I, wobei R³ Wasserstoff bedeutet, mit einem elektrophilen Aminierungsreagenz in Gegenwart einer Base:

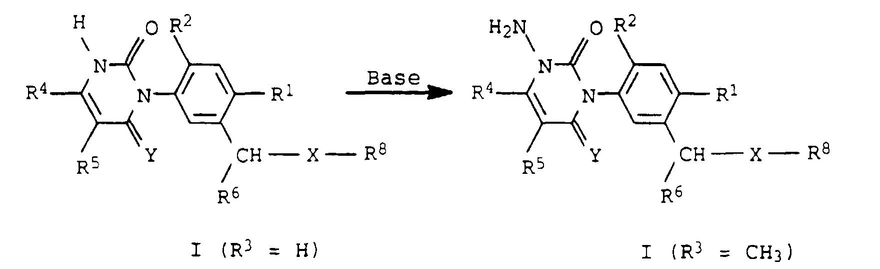

Als Aminierungsreagenz hat sich bisher 2,4-Dinitrophenoxyamin besonders bewährt, jedoch kann z.B. auch Hydroxylamin-O-sulfonsäure (HOSA) verwendet werden, die aus der Literatur bereits als Aminierungsreagenz bekannt ist (vgl. z.B. E. Hofer et al., Synthesis 1983, 466; W. Friedrichsen et al., Heterocycles 20 (1983) 1271; H. Hart et al., Tetrahedron Lett. 25 (1984) 2073; B. Vercek et al., Monatsh. Chem. 114 (1983) 789; G. Sosnousky et al., Z. Naturforsch. 38 (1983) 884; R.S. Atkinson et al., J. Chem. Soc. Perkin Trans. 1987, 2787).

Die Aminierung kann auf an sich bekannte Weise durchgeführt werden (siehe z.B. T. Sheradsky, Tetrahedron Lett. 1968, 1909; M.P. Wentland et al., J. Med. Chem. 27 (1984) 1103 und insbesondere EP-A 240 194, EP-A 476 697, EP-A 517 181 und WO 95/06641, wo die Aminierung von Uracilen gelehrt wird).

Normalerweise führt man die Umsetzung in einem polaren Lösungsmittel durch, z.B. in Dimethylformamid, N-Methylpyrrolidon, Dimethylsulfoxid oder in Ethylacetat, das sich bisher als besonders geeignet erwiesen hat.

Als Base eignen sich beispielsweise Alkalimetallcarbonate wie Kaliumcarbonat, Alkalimetallalkoholate wie Natriummethylat und Kalium-tert.-butanolat oder Alkalimetallhydride wie Natriumhydrid.

Die Menge an Base und Aminierungsmittel liegt vorzugsweise jeweils bei der 0,5- bis zweifachen molaren Menge, bezogen auf die Menge an Ausgangsverbindung.

Je nach Bedeutung von R⁷ und R⁸ kann es erforderlich sein, diesen Substituenten vor der Aminierung in an sich bekannter Weise zu schützen. Dies ist besonders empfehlenswert, wenn R⁷ oder R⁸ Wasserstoff ist.

### Verfahren D)

Schwefelung eines Benzylhydroxylamins der Formel I mit Y = Sauerstoff:

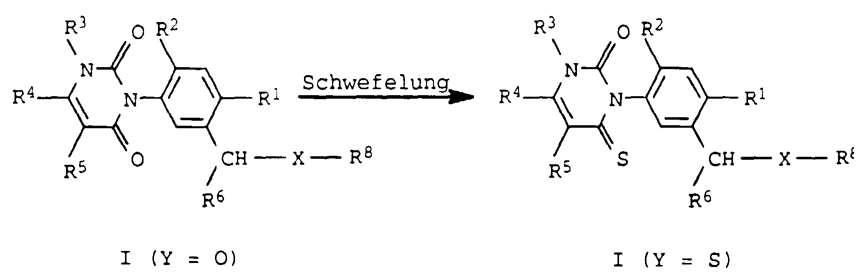

Die Schwefelung erfolgt in der Regel in einem inerten Lösungs- oder Verdünnungsmittel, beispielsweise in einem aromatischen Kohlenwasserstoff wie Toluol und den Xylolen, in einem Ether wie Diethylether, 1,2-Dimethoxyethan und Tetrahydrofuran, oder in einem organischen Amin wie Pyridin.

Als Schwefelungsreagenz eignen sich besonders gut Phosphor(V)-sulfid und 2,4-Bis(4-methoxyphenyl)-1,3,2,4-dithiadiphosphetan-2,4-dithion ("Lawesson-Reagenz").

Üblicherweise ist die 1- bis 5-fache molare Menge, bezogen auf die zu schwefelnde Ausgangsverbindung, für eine weitgehend vollständige Umsetzung ausreichend.

Die Reaktionstemperatur liegt normalerweise bei 20 bis 200°C, vorzugsweise bei 40°C bis zur Siedetemperatur des Reaktionsgemisches.

### Verfahren E)

Alkylierung oder Acylierung eines Benzylhydroxylamins der Formel I, bei dem R⁷ Wasserstoff bedeutet, in Gegenwart einer Base:

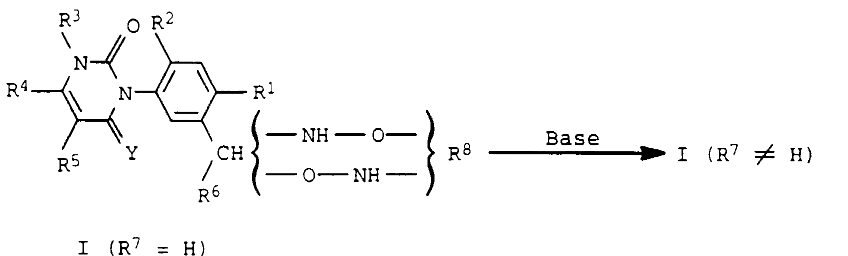

Die Alkylierung kann beispielsweise mit dem Halogenid, vorzugsweise dem Chlorid oder Bromid, dem Sulfat, Sulfonat, vorzugsweise dem Methansulfonat (Mesylat), Benzolsulfonat, p-Toluolsulfonat (Tosylat), p-Brombenzolsulfonat (Brosylat), dem Trifluormethansulfonat (Triflat) oder der Diazoverbindung eines unsubstituierten oder substituierten Alkans, Cycloalkans, Halogenalkans, Alkens oder Alkins vorgenommen werden.

Geeignete Acylierungsmittel sind z.B. die Säurehalogenide, insbesondere die Säurechloride, die Anhydride, Isocyanate und Sulfonylchloride von gegebenenfalls substituierten Alkan-, Cycloalkan-, Alken-, Alkin- oder Phenylcarbonsäuren. Es kommen aber auch die freien Säuren oder deren Anhydride in Betracht, sofern dann in Gegenwart eines Kondensationsmittels wie Carbonyldiimidazol und Dicyclohexylcarbodiimid gearbeitet wird.

Normalerweise arbeitet man in einem inerten organischen Lösungsmittel, vorzugsweise in einem aprotische Lösungsmittel, z.B. einem aliphatische oder cyclischen Ether wie 1,2-Dimethoxyethan, Tetrahydrofuran und Dioxan, einem aliphatischen Keton wie Aceton, einem Amid wie Dimethylformamid, einem Sulfoxid wie Dimethylsulfoxid, einem Harnstoff wie Tetramethylharnstoff und 1,3-Dimethyltetrahydro-2(1H)-pyrimidinon, einem Carbonsäureester wie Essigsäurethylester, oder einem halogenierten aliphatischen oder aromatischen Kohlenwasserstoff wie Dichlormethan und Chlorbenzol.

Als Base eignen sich sowohl anorganische Basen, z.B. Alkalimetallcarbonate wie Natriumcarbonat und Kaliumcarbonat, Alkalimetallhydrogencarbonate wie Natrium- und Kaliumhydrogencarbonat, oder Alkalimetallhydride wie Natriumhydrid und Kaliumhydrid, als auch organische Basen, z.B. Amine wie Triethylamin, Pyridin und N,N-Diethylanilin, oder Alkalimetallalkoholate wie Natriummethanolat, -ethanolat und Kalium-tert.-butanolat.

Die Menge an Base und Alkylierungsmittel liegt vorzugsweise bei der 0,5- bis zweifachen molaren Menge, bezogen auf die Menge an I mit R⁷ = Wasserstoff.

Im allgemeinen empfiehlt sich eine Reaktionstemperatur von 0°C bis zur Siedetemperatur des Reaktionsgemisches, insbesondere von 0 bis 60°C.

Etwaige Regioselektivitätsprobleme bei Ausgangsverbindungen mit R³ = Wasserstoff können auf an sich bekannte Weise (Verwendung von 2 Äquivalenten Base, Einführung einer Schutzgruppe etc.) vermieden werden.

### Verfahren F)

Reduktion einer Oximinoverbindung der Formel VII:

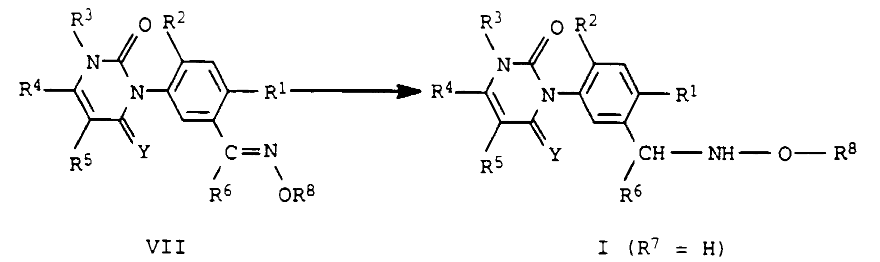

Als Reduktionsmittel eignen sich beispielsweise Hydride wie Borankomplexe, z.B. Borandimethylsulfid oder Boran-Pyridin-Komplexe, des weiteren Silane wie Triethylsilan und Diphenylmethylsilan, oder molekularer Wasserstoff unter Verwendung eines Katalysators wie Platin auf Kohle.

Bei der Verwendung von Wasserstoff empfiehlt sich eine Reaktionsführung unter sauren Bedingungen, z.B. in einer organischen oder anorganischen Säure als Lösungsmittel.

Der Wasserstoffdruck liegt üblicherweise bei Normaldruck bis etwa 10 bar Überdruck.

Im allgemeinen gelingt die Reduktion bei Temperaturen von (-5) bis +50°C.

Die Menge an Reduktionsmittel ist nicht kritisch. Es kann mit einer geringeren Menge an Reduktionsmittel, bezogen auf die Menge an VII, oder mit einem Überschuß, bis etwa zur 15fachen molaren Menge, gearbeitet werden. Vorzugsweise verwendet man die 0,5- bis zweifache molare Menge an Reduktionsmittel, bezogen auf die Oximinoverbindung VII.

### Verfahren G)

Spaltung einer Alkylidenaminoxy-Verbindung der Formel VIII:

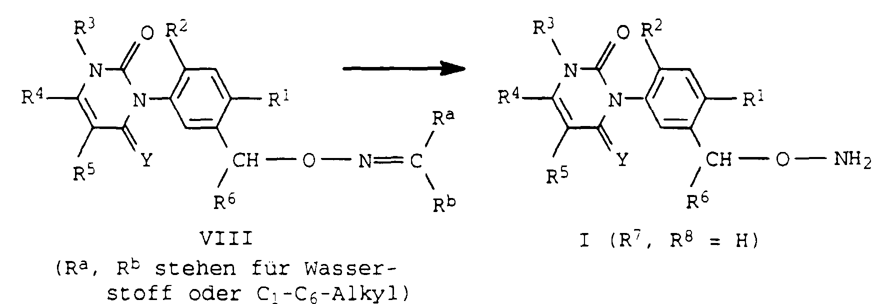

Die Spaltung wird zweckmäßig mit einer Brønsted-Säure als Katalysator durchgeführt. Normalerweise ist dabei die 0,5-bis 2fache molare Menge an Säure, bezogen auf die Menge an VIII, ausreichend. In Betracht kommen z.B. organische Säuren wie Essigsäure und anorganische Säuren wie Schwefelsäure und Salzsäure.

Die Reaktion kann direkt in der Säure oder in einem inerten Lösungsmittel, z.B. in Toluol, durchgeführt werden.

Vorzugsweise führt man die Spaltung in Gegenwart eines Oxims wie Hydroxylamin und Methylhydroxylamin durch, wobei das Oxim dann etwa äquimolar oder im Überschuß bis zur 20fachen molaren Menge, bezogen auf die Menge an VIII, eingesetzt wird.

Im allgemeinen empfiehlt sich eine Reaktionstemperatur von 0°C bis zur Siedetemperatur des Reaktionsgemisches.

Die Alkylidenaminoxy-Verbindungen VIII sind ihrerseits z.B. aus Benzylderivaten IX erhältlich:

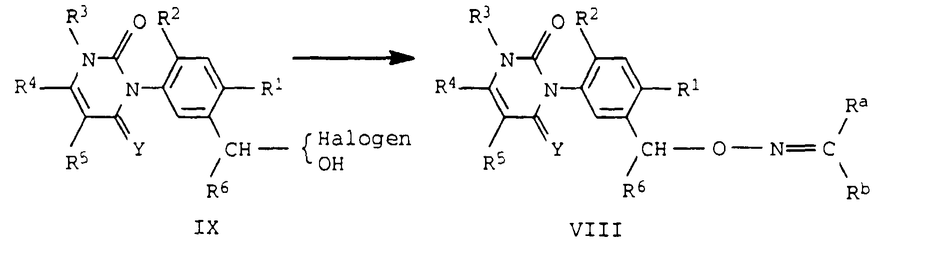

Die Umsetzung erfolgt in der Regel in einem inerten Lösungs-/Verdünnungsmittel mit dem gewünschten Alkylidenaminohydroxid HO-N=C(R^{a},R^{b}). Wird IX als Benzylhalogenid (IXa) eingesetzt, so empfiehlt sich das Arbeiten in Gegenwart einer organischen Base wie Triethylamin oder einer anorganischen Base wie Natrium- und Kaliumcarbonat. Geht man dagegen von einem Benzylalkohol (IXb) aus, so wird ein Kondensationshilfsmittel wie Carbodiimidazol benötigt.

Als Lösungsmittel kommen beispielsweise Aromaten wie Toluol und die Xylole, Ester wie Essigsäureethylester, Ether wie Diethylether und Tetrahydrofuran, halogenierte Aliphaten wie Methylenchlorid oder basische Lösungsmittel wie Pyridin und Dimethylformamid in Betracht.

Zweckmäßigerweise werden IX und Alkylidenaminohydroxid in etwa stöchiometrischen Mengen eingesetzt, oder man arbeitet mit einem geringen Uberschuß der einen oder anderen Komponente, bis etwa 10 mol-%.

Im allgemeinen erfolgt die Reaktionsführung bei einer Temperatur von 0°C bis Siedetemperatur des Reaktionsgemisches.

### Verfahren H)

Umsetzung von Benzylalkoholen IXa mit Hydroxylaminen HO-N(R⁷)-R⁸:

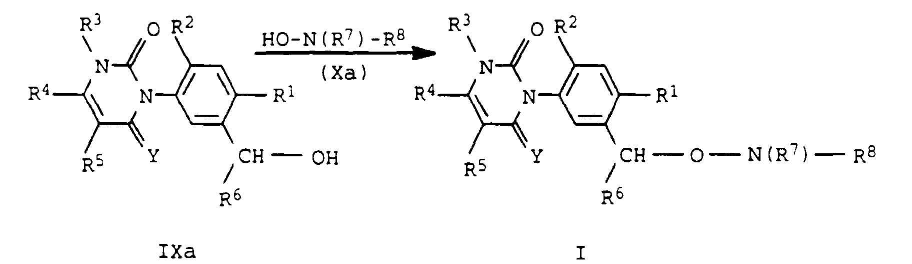

Vorzugsweise arbeitet man in Gegenwart eines Kondensationshilfsmittels wie Carbodiimidazol, dessen Menge normalerweise bei der 0,5- bis 2fachen molaren Menge, bezogen auf die Menge an IXa, liegt.

Die Reaktion wird in der Regel in einem inerten organischen Lösungsmittel, z.B. einem Kohlenwasserstoff wie Toluol und die Xylole, einem Ester wie Essigsäureethylester oder einem Ether wie Diethylether und Tetrahydrofuran durchgeführt.

Zweckmäßig setzt man IXa und Xa in etwa stöchiometrischen Mengen ein, oder man arbeitet mit einem geringen Überschuß der einen oder anderen Komponente, bis etwa 10 mol-%.

Im allgemeinen empfiehlt sich eine Reaktionstemperatur von 0°C bis zur Siedetemperatur des Reaktionsgemisches.

### Verfahren K)

Halogenierung eines Benzylhydroxylamins der Formel I, bei dem R⁵ Wasserstoff bedeutet:

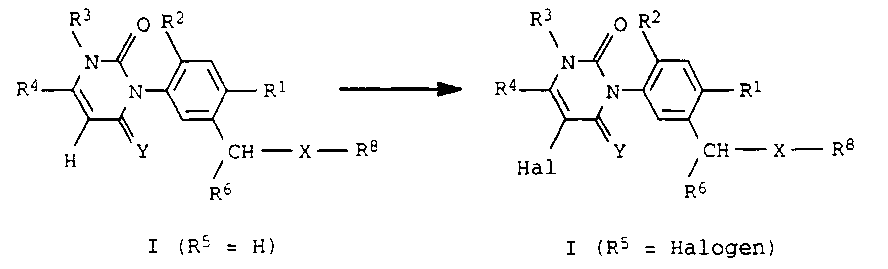

Die Halogenierung erfolgt in der Regel in einem inerten organischen Lösungs- oder Verdünnungsmittel. Für die Chlorierung und Bromierung kommen beispielsweise aliphatische Carbonsäuren wie Essigsäure, oder chlorierte aliphatische Kohlenwasserstoffe wie Methylenchlorid, Chloroform und Tetrachlorkohlenstoff, in Betracht. Für die Iodierung sind niedrig siedende aliphatischen Carbonsäuren wie Essigsäure besonders bevorzugt.

Für die Chlorierung und Bromierung eignen sich besonders elementares Chlor bzw. Brom, oder Sulfurylchlorid bzw. Sulfurylbromid, bei einer Reaktionstemperatur von vorzugsweise 0 bis 60°C, insbesondere 10 bis 30°C.

Gewünschtenfalls kann die Chlorierung und Bromierung in Gegenwart eines säurebindenden Mittels erfolgen, wobei Natriumacetat und tertiäre Amine wie Triethylamin, Dimethylanilin und Pyridin besonders bevorzugt sind.

Als Iodierungsmittel ist elementares Iod besonders bevorzugt, wobei in diesem Fall die Reaktionstemperatur bei ca. 0 bis 110°C, vorzugsweise bei 10 bis 30°C, liegt.

Besonders vorteilhaft verläuft die Iodierung in Gegenwart einer Mineralsäure wie rauchender Salpetersäure.

Die Menge an Halogenierungsmittel ist nicht kritisch; normalerweise verwendet man äquimolare Mengen an Halogenierungsmittel oder einen Überschuß bis etwa 200 mol-%, bezogen auf die Ausgangsverbindung (I mit R⁵ = Wasserstoff).

Überschüssiges Iod kann beispielsweise nach der Reaktion mittels gesättigter wäßriger Natriumhydrogensulfit-Lösung entfernt werden.

### Verfahren L)

Alkylierung eines Hydroxylamins oder einer Hydroxamsäure mit einem Benzylhalogenid oder Benzylalkoholderivat der Formel XI in Gegenwart einer Base:

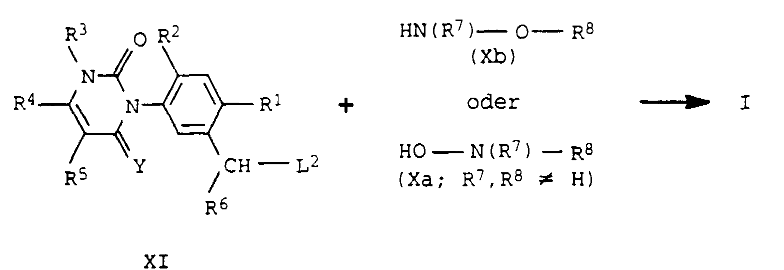

L² bedeutet Halogen oder Niederalkylsulfonat, Niederhalogenalkylsulfonat oder Phenylsulfonat, das substituiert sein kann.

In der Regel arbeitet man in einem inerten organischen Lösungsmittel, wobei besonders aprotische Lösungsmittel, z.B. aliphatische oder cyclische Äther wie vorzugsweise 1,2-Dimethoxyethan, Tetrahydrofuran und Dioxan, aliphatische Ketone wie vorzugsweise Aceton, Amide wie vorzugsweise Dimethylformamid, Sulfoxide wie vorzugsweise Dimethylsulfoxid, Harnstoffe wie Tetramethylharnstoff und 1,3-Dimethyltetrahydro-2(1H)-pyrimidinon, Carbonsäureester wie Essigsäureethylester, oder halogenierte aliphatische oder aromatische Kohlenwasserstoffe wie Dichlormethan und Chlorbenzol in Betracht kommen.

Als Base eignen sich sowohl anorganische Basen, z.B. Carbonate wie die Alkalimetallcarbonate, insbesondere Natriumcarbonat und Kaliumcarbonat, Hydrogencarbonate wie die Alkalimetallhydrogencarbonate, insbesondere Natrium- und Kaliumhydrogencarbonat, oder Alkalimetallhydride wie Natriumhydrid und Kaliumhydrid, als auch organische Basen, z.B. Amine wie Triethylamin, Pyridin und N,N-Diethylanilin, oder Alkalimetallalkoholate wie Natriummethanolat, Natriumethanolat und Kalium-tert.-butanolat.

Die Mengen an Base und Alkylierungsmittel XI liegen normalerweise jeweils bei der 0,5- bis 2-fachen molaren Menge, bezogen auf die Menge an X.

Im allgemeinen arbeitet man bei einer Reaktionstemperatur von (-78)°C bis zur Siedetemperatur des Reaktionsgemisches, insbesondere bei (-60) bis 60°C.

Die Enaminester der Formel IV sind neu. Ihre Herstellung kann nach an sich bekannten Methoden erfolgen, z. B. nach einem der folgenden Verfahren:

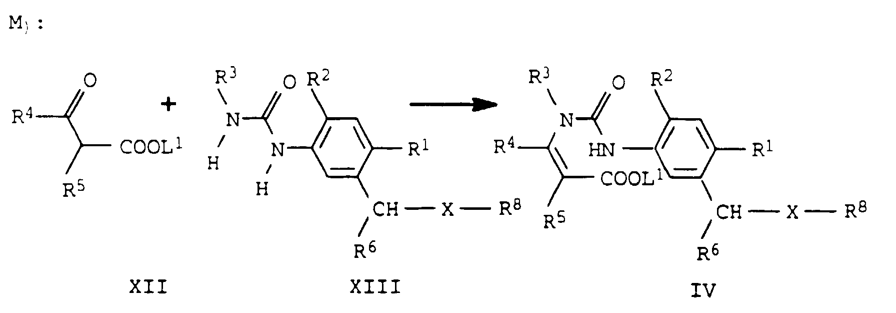

Vorzugsweise arbeitet man im wesentlichen wasserfrei in einem inerten Lösungs- oder Verdünnungsmittel, besonders bevorzugt in Gegenwart eines sauren oder basischen Katalysators.

Als Lösungs- oder Verdünnungsmittel kommen insbesondere mit Wasser azeotrop mischbare organische Lösungsmittel, beispielsweise Aromaten wie Benzol, Toluol und die Xylole, halogenierte Kohlenwasserstoffe wie Methylenchlorid, Chloroform, Tetrachlorkohlenstoff und Chlorbenzol, aliphatische und cyclische Ether wie 1,2-Dimethoxyethan, Tetrahydrofuran und Dioxan, oder Cyclohexan, aber auch Alkohole wie Methanol und Ethanol, in Betracht.

Als saure Katalysatoren eignen sich bevorzugt starke Mineralsäuren wie Schwefelsäure und Salzsäure, Phosphor enthaltende Säuren wie Orthophosphorsäure und Polyphosphorsäure, organische Säuren wie p-Toluolsulfonsäure sowie saure Kationenaustauscher wie "Amberlyst 15" (Fa. Fluka).

Als basische Katalysatoren eignen sich z.B. Metallhydride wie Natriumhydrid sowie besonders bevorzugt Metallalkoholate wie Natriummethanolat und Ethanolat.

Zweckmäßig setzt man XIII und den β-Ketoester XII in etwa stöchiometrischen Mengen ein, oder man arbeitet mit einem geringen Überschuß der einen oder anderen Komponente, bis etwa 10 mol-%.

Normalerweise ist die 0,5- bis 2fache molare Menge an Katalysator, bezogen auf die Menge einer der Ausgangsverbindungen, ausreichend.

Im allgemeinen erfolgt die Reaktionsführung bei einer Temperatur von 60 bis 120°C, zur raschen Entfernung von entstehendem Wasser vorzugsweise bei der Siedetemperatur des Reaktionsgemisches.

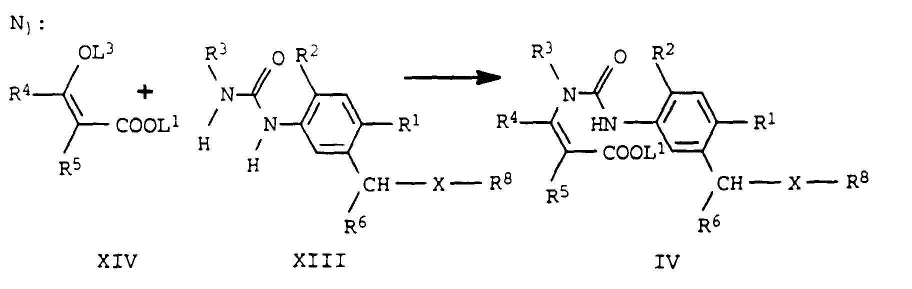

L³ steht für C₁-C₄-Alkyl oder Phenyl.

Diese Umsetzung kann beispielsweise in einem inerten, mit Wasser mischbaren, organischen Lösungsmittel, beispielsweise einem aliphatischen oder cyclischen Ether wie 1,2-Dimethoxyethan, Tetrahydrofuran und Dioxan, oder einem niederen Alkohol, insbesondere Ethanol, durchgeführt werden, wobei die Reaktionstemperatur normalerweise bei 50 bis 100°C, vorzugsweise bei der Siedetemperatur des Reaktionsgemisches, liegt.

Die Reaktion kann jedoch auch in einem aromatischen Verdünnungsmittel wie Benzol, Toluol und den Xylolen durchgeführt werden, wobei in diesem Fall der Zusatz entweder eines sauren Katalysators wie Salzsäure und p-Toluolsulfonsäure oder einer Base, z.B. eines Alkalimetallalkoholates wie Natriummethanolat und Natriumethanolat, empfehlenswert ist. Auch bei dieser Verfahrensvariante liegt die Reaktionstemperatur normalerweise bei 50 bis 100°C, bevorzugt jedoch bei 60 bis 80°C.

Bezüglich der Mengenverhältnisse gelten die Angaben für Methode M).

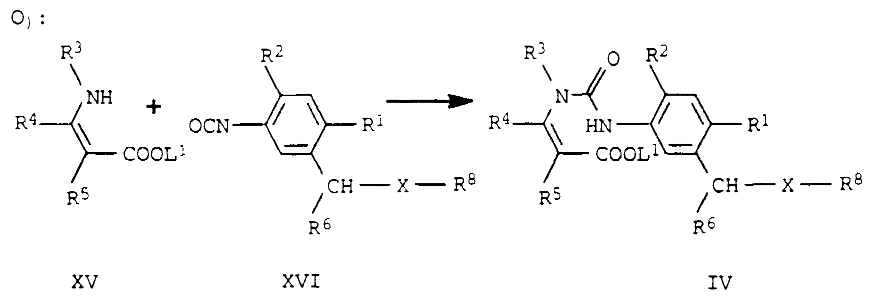

Die Umsetzung erfolgt zweckmäßig in Gegenwart eines im wesentlichen wasserfreien aprotischen organischen Lösungs- oder Verdünnungsmittels, beispielsweise eines aliphatischen oder cyclischen Ethers wie Diethylether, 1,2-Dimethoxyethan, Tetrahydrofuran und Dioxan, eines aliphatischen oder aromatischen Kohlenwasserstoffs wie n-Hexan, Benzol, Toluol und den Xylolen, eines halogenierten, aliphatischen Kohlenwasserstoffs wie Methylenchlorid, Chloroform, Tetrachlorkohlenstoff, 1,2-Dichlorethan und Chlorbenzol, eines aprotischen, polaren Lösungsmittel wie Dimethylformamid, Hexamethylphosphorsäuretriamid und Dimethylsulfoxid, oder eines Gemisches aus den genannten Solventien.

Gewünschtenfalls kann auch in Gegenwart einer Metallhydridbase wie Natrium- und Kaliumhydrid, eines Alkalimetall- oder Erdalkalimetallalkoholates wie Natriummethanolat, -ethanolat und Kalium-tert.-butanolat, oder einer organischen tertiären Base wie Triethylamin und Pyridin gearbeitet werden, wobei die organische Base gleichzeitig als Lösungsmittel dienen kann.

Zweckmäßig setzt man die Ausgangsverbindungen in stöchiometrischen Mengen ein oder man arbeitet mit einem geringen Überschuß der einen oder anderen Komponente bis etwa 10 mol-%. Beim Arbeiten ohne Lösungsmittel in Gegenwart einer organischen Base empfiehlt es sich, letztere in einem größeren Überschuß einzusetzen.

Normalerweise ist eine Reaktionstemperatur von (-80) bis 50°C, insbesondere (-60) bis 30°C ausreichend.

In einer besonders bevorzugten Ausführungsform wird der erhaltene Enaminester IV mit überschüssiger Base direkt (d.h. "in situ") gemäß Verfahren A) in das entsprechende Wertprodukt I übergeführt. Etwaige Nebenprodukte (z.B. C-Alkylierungsprodukte bei Verbindungen mit R⁵ = Wasserstoff), lassen sich mittels üblicher Trennverfahren wie Kristallisation und Chromatographie entfernen.

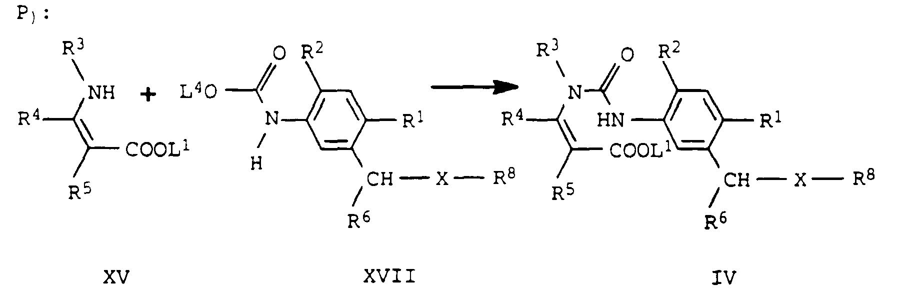

L⁴ steht für C₁-C₄-Alkyl oder Phenyl.

Diese Reaktion erfolgt zweckmäßig in einem aprotischen, polaren Lösungs- oder Verdünnungsmittel wie Dimethylformamid, 2-Butanon, Dimethylsulfoxid und Acetonitril, und zwar vorteilhaft in Gegenwart einer Base, beispielsweise eines Alkalimetall- oder Erdalkalimetallalkoholats, insbesondere eines Natriumalkanolates wie Natriummethanolat, eines Alkalimetall- oder Erdalkalimetallcarbonates, insbesondere Natriumcarbonat, oder eines Alkalimetallhydrids wie Lithium- und Natriumhydrid.

Normalerweise ist die 0,5- bis 2fache molare Menge an Base, bezogen auf die Menge von XV oder XVII, ausreichend.

Die Reaktionstemperatur liegt im allgemeinen bei 80 bis 180°C, vorzugsweise bei der Siedetemperatur des Reaktionsgemisches.

Bezüglich der Mengenverhältnisse der Ausgangsverbindungen gelten die Angaben für Methode M).

In einer besonders bevorzugten Ausführungsform verwendet man ein Natriumalkoholat als Base und destilliert den im Laufe der Reaktion entstehenden Alkohol kontinuierlich ab. Die auf diese Weise hergestellten Enaminester IV können ohne Isolierung aus der Reaktionsmischung gemäß Verfahren A) zu einem Salz des entsprechenden Benzylhydroxylamins I cyclisiert werden.

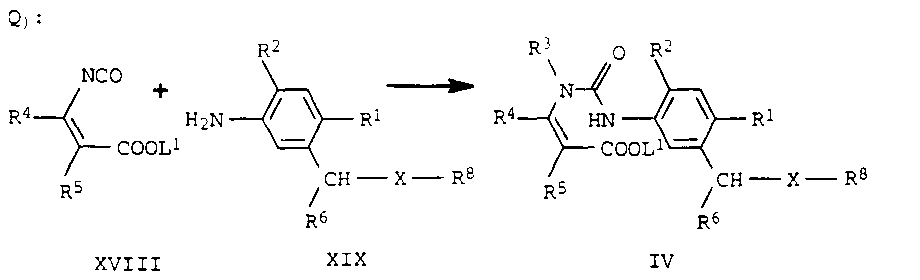

Diese Umsetzung erfolgt zweckmäßig in einem im wesentlichen wasserfreien, aprotischen, organischen Lösungs- oder Verdünnungsmittel, beispielsweise in Gegenwart eines aliphatischen oder cyclischen Ethers wie Diethylether, 1,2-Dimethoxyethan, Tetrahydrofuran und Dioxan, eines aliphatischen oder aromatischen Kohlenwasserstoffs wie n-Hexan, Benzol, Toluol und den Xylolen, eines halogenierten, aliphatischen Kohlenwasserstoffs wie Methylenchlorid, Chloroform, Tetrachlorkohlenstoff, 1,2-Dichlorethan und Chlorbenzol, eines aprotischen, polaren Lösungsmittels wie Dimethylformamid, Hexamethylphosphorsäuretriamid und Dimethylsulfoxid, oder eines Gemisches aus den genannten Solventien.

Gewünschtenfalls kann in Gegenwart einer Metallhydridbase wie Natrium- und Kaliumhydrid, eines Alkalimetall- oder Erdalkalimetallalkoholates wie Natriummethanolat, -ethanolat und Kalium-tert.-butanolat, oder einer organischen Stickstoffbase wie Triethylamin und Pyridin gearbeitet werden, wobei die organische Base gleichzeitig als Lösungsmittel dienen kann.

Zweckmäßigerweise setzt man die Ausgangsverbindungen in stöchiometrischen Mengen ein oder man verwendet eine der Komponenten im Überschuß, bis etwa 20 mol-%. Beim Arbeiten ohne Lösungsmittel in Gegenwart einer organischen Base verwendet man letztere vorteilhaft in einem noch größeren Überschuß.

Die Reaktionstemperatur liegt im allgemeinen bei (-80) bis 150°C, vorzugsweise bei (-30)°C bis Siedepunkt des jeweiligen Reaktionsgemisches.

Die Enamincarboxylate der Formel V sind ebenfalls neu; auch sie können auf an sich bekannte Weise hergestellt werden, beispielsweise aus einem Anilinderivat der Formel XV nach folgendem Reaktionsschema R):

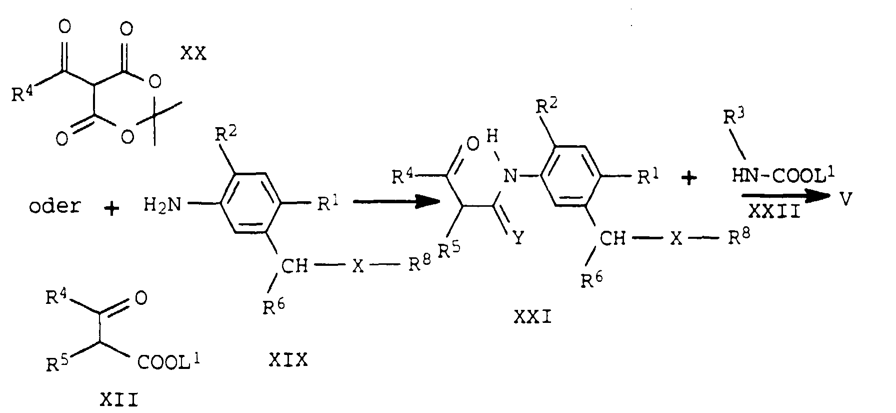

Die Umsetzung von XX mit XIX erfolgt vorzugsweise in einem wasserfreien inerten aprotischen Lösungsmittel, beispielsweise in einem halogenierten Kohlenwasserstoff wie Methylenchlorid, Chloroform, Tetrachlorkohlenstoff und Chlorbenzol, einem aromatischen Kohlenwasserstoff wie Benzol, Toluol und den Xylolen, oder einem aliphatischen oder cyclischen Ether wie Diethylether, Dibutylether, 1,2-Dimethoxyethan, Tetrahydrofuran und Dioxan.

Die Reaktionstemperatur liegt bei dieser Umsetzung (von XX mit XIX) im allgemeinen bei etwa 70 bis 140°C, insbesondere bei 100 bis 120°C.

Bei der Umsetzung von XII mit XIX handelt es sich um eine Aminolyse, die in der Regel entweder ohne Lösungsmittel [vgl. z.B. J. Soc. Dyes Col. 42, 81 (1926), Ber. 64, 970 (1931); Org. Synth., Coll. Vol. IV, 80 (1963) und J. Am. Chem. Soc. 70, 2402 (1948)] oder in einem inerten wasserfreien Lösungs-/Verdünnungsmittel, insbesondere in einem aprotischen Solvens, beispielsweise in einem Aromaten wie Toluol und den Xylolen, oder einem halogenierten Aromaten wie Chlorbenzol, durchgeführt wird.

Hierbei empfiehlt sich das Arbeiten in Gegenwart eines basischen Katalysators, beispielsweise eines höher siedenden Amins [siehe z. B. Helv. Chim. Acta 11, 779 (1928) und U.S. 2,416,738] oder von Pyridin.

Vorzugsweise liegt die Reaktionstemperatur bei ca. 20 bis 160°C.

Zweckmäßigerweise setzt man die Ausgangsverbindungen jeweils in etwa stöchiometrischen Mengen ein oder man arbeitet mit einem geringen Überschuß der einen oder anderen Komponente, bis etwa 10 mol-%. Arbeitet man in Gegenwart eines basischen Katalysators, so ist normalerweise die 0,5- bis 2fache molare Menge an Katalysator, bezogen auf die Menge eines der Edukte, ausreichend.

Die anschließende Umsetzung der so hergestellten Verbindungen der Formel XXI mit dem Amin HN(R³)-COO^{L1} wird vorteilhaft in einem weitgehend wasserfreien Lösungs-/Verdünnungsmittel bei Normaldruck durchgeführt, besonders bevorzugt in Gegenwart eines sauren Katalysators.

Zur Herstellung von Enamincarboxylaten V mit R³ = Amino empfiehlt es sich, Verbindungen XXII mit geschützter Aminogruppe (z.B. als Hydrazon) einzusetzen.

Als Lösungs-/Verdünnungsmittel kommen insbesondere mit Wasser azeotrop mischbare organische Flüssigkeiten, beispielsweise Aromaten wie Benzol, Toluol und die Xylole, oder halogenierte Kohlenwasserstoffe wie Tetrachlorkohlenstoff und Chlorbenzol, in Betracht.

Geeignete Katalysatoren sind insbesondere starke Mineralsäuren wie Schwefelsäure, organische Säuren wie p-Toluolsulfonsäure, Phosphor enthaltende Säuren wie Orthophosphorsäure und Polyphosphorsäure oder saure Kationenaustauscher wie "Amberlyst 15" (Fa. Fluka).

Im allgemeinen liegt die Reaktionstemperatur bei etwa 70 bis 150°C; zur raschen Entfernung des entstehenden Reaktionswassers arbeitet man jedoch zweckmäßigerweise bei der Siedetemperatur des jeweiligen Reaktionsgemisches.

Die Verbindungen der Formeln XIII, XVI, XVII und XXI sind ebenfalls neu. Auch sie lassen sich auf an sich bekannte Weise herstellen, besonders vorteilhaft aus den Verbindungen der Formel XIX:

### S) "Phosgenierung" von Verbindungen der Formel XIX und Hydrolyse der Verfahrensprodukte XVI mit Ammoniak(derivaten):

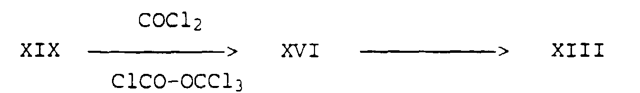

Das Verfahren kann in einem inerten, im wesentlichen wasserfreien Lösungs-/Verdünnungsmittel oder ohne Solvens durchgeführt werden. Die Überführung der Amino- in die Isocyanatgruppe erfolgt dabei vorzugsweise mittels Phosgen oder Chlorameisensäuretrichlormethylester.

Als Lösungs-/Verdünnungsmittel kommen insbesondere aprotische, organische Solventien, beispielsweise Aromaten wie Toluol und die Xylole, halogenierte Kohlenwasserstoffe wie Methylenchlorid, Chloroform, 1,2-Dichlorethan und Chlorbenzol, aliphatische oder cyclische Ether wie 1,2-Dimethoxyethan, Tetrahydrofuran und Dioxan, Ester wie Essigsäureethylester, sowie Gemische dieser Lösungsmittel in Betracht.

Abhängig vom eingesetztem Anilinderivat XIX kann der Zusatz einer Base wie Triethylamin vorteilhaft sein, beispielsweise in 0,5- bis 2-facher molarer Menge, bezogen auf die Menge an XIX.

Die Phenylisocyanate XVI entstehen üblicherweise bei Reaktionstemperaturen von 50°C bis Siedetemperatur des Reaktionsgemisches; sie können anschließend mit Ammoniak oder einem reaktiven Derivat des Ammoniaks zu den Phenylharnstoffderivaten XIII umgesetzt werden.

### ^{T}) Umsetzung mit Alkalimetallcyanaten:

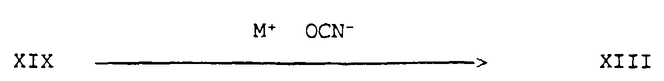

M⁺ steht für das Äquivalent eines Metallions, insbesondere für ein Alkalimetallion wie Natrium und Kalium.

Die Umsetzung erfolgt in der Regel in einem inerten Lösungs-/ Verdünnungsmittel, beispielsweise in einem aromatischen Kohlenwasserstoff wie Toluol und den Xylolen, in einem aliphatischen oder cyclischen Ether wie Tetrahydrofuran und Dioxan, in einem niederen Alkohol wie Methanol und Ethanol, in Wasser oder in einem Gemisch der genannten Solventien.

Die Menge an Cyanat ist nicht kritisch; für eine vollständige Umsetzung benötigt man mindestens äquimolare Mengen an Anilin-Verbindung XIX und Cyanat, jedoch kann auch ein Überschuß an Cyanat, bis etwa 100 mol-%, Vorteile bieten.

Die Reaktionstemperatur liegt im allgemeinen bei 0°C bis Siedetemperatur des Reaktionsgemisches.

### U) Umsetzung mit Estern L⁴O-CO-L⁵:

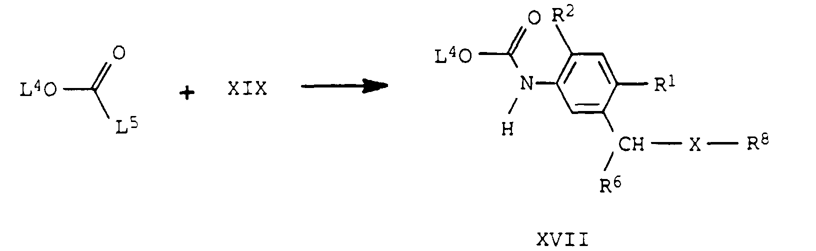

L⁵ steht für Halogen, vorzugsweise Chlor oder Brom, für C₁-C₄-Alkoxy oder für Phenoxy.

Als Lösungs-/Verdünnungsmittel eignen sich beispielsweise aromatische Kohlenwasserstoffe wie Toluol und die Xylole, halogenierte Kohlenwasserstoffe wie Methylenchlorid, Chloroform, 1,2-Dichlorethan und Chlorbenzol, aliphatische oder cyclische Ether wie 1,2-Dimethoxyethan, Tetrahydrofuran und Dioxan, Ester wie Essigsaureethylester, Alkohole wie Methanol und Ethanol, oder Wasser.
Auch Gemische aus einem organischen Lösungsmittel und Wasser kommen in Betracht.

Bevorzugt arbeitet man in Gegenwart einer Base, z.B. in Gegenwart eines Alkalimetallhydroxides, -carbonates oder -alkoholates wie Natriumhydroxid, -carbonat, -methanolat und -ethanolat, oder eines tertiären Amins wie Pyridin und Triethylamin.

Gewünschtenfalls kann auch ein Katalysator, z.B. eine Lewissäure wie Antimontrichlorid, zugesetzt werden.

Zweckmäßigerweise setzt man die Ausgangsverbindungen und die Base in etwa stöchiometrischen Mengen ein, jedoch kann auch die eine oder andere Komponente im Überschuß, bis ca. 100 mol-%, vorliegen.

Die Menge an Katalysator beträgt im allgemeinen 1 bis 50 mol-%, bevorzugt 2 bis 30 mol-%, bezogen auf die Menge an eingesetztem Anilin-Verbindung XIX.

Die Umsetzung gelingt normalerweise bei Reaktionstemperaturen von (-40)°C bis zur Siedetemperatur des Reaktionsgemisches.

Die Anilin-Verbindungen der Formel XIX sind ebenfalls neu. Ihre Synthese erfolgt üblicherweise aus den entsprechende Nitro-Veri bindungen XXIII durch Hydrierung oder aus Anilinen der Formel XXIV, die gemäß Verfahren F) in die entsprechenden Anilin-Verbindungen der Formel XIX übergeführt werden können:

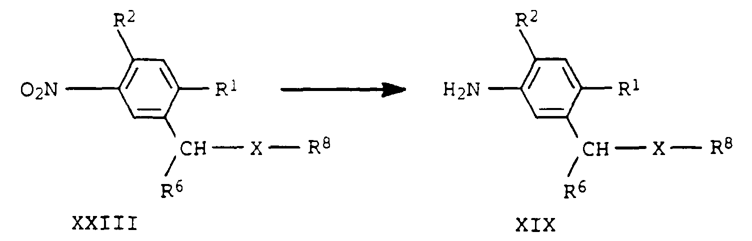

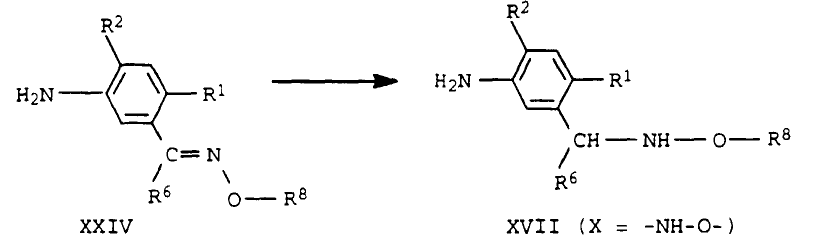

Auch die Nitro-Verbindungen der Formel XXIII sind neu. Sie lassen sich nach an sich bekannten Methoden, z.B. durch Nitrierung der entsprechenden Benzylamine, darstellen.

Die Verbindungen der Formel VII, X, XI, XII, XIV, XV, XVIII, XIX und XXIV sind bekannt oder auf an sich bekannte Weise darstellbar (vgl. z.B. WO 92/02088 und DE-A 42 37 920).

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Je nach Substitutionsmuster der Zielverbindungen kann es empfehlenswert sein, die Reihenfolge einzelner Reaktionsschritte zu vertauschen, damit bestinmmte Nebenprodukte nicht oder in geringerer Menge anfallen.

Die Aufarbeitung der Reaktionsgemische erfolgt in der Regel nach an sich bekannten Methoden, beispielsweise durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die Benzylhydroxylamine der Formel I können ein oder mehrere Chiralitätszentren enthalten und fallen dann üblicherweise als Enantiomeren- oder Diastereomerengemische an, sofern keine gezielte Synthese auf ein Isomeres hin vorgenommen wird. Die Mischungen können gewünschtenfalls nach den hierfür üblichen Methoden, z.B. mittels Kristallisation oder Chromatographie an einem optisch aktiven Adsorbat, in die weitgehend reinen Isomeren getrennt werden. Reine optisch aktive Isomere lassen sich beispielsweise auch aus entsprechenden optisch aktiven Ausgangsmaterialien herstellen.

Benzylhydroxylamine I, bei denen R³, R⁷ oder R⁸ Wasserstoff bedeutet, lassen sich auf an sich bekannte Weise in ihre Salze, vorzugsweise in ihre Alkalimetallsalze, überführen.

Salze von I, deren Metallion kein Alkalimetallion ist, können durch Umsalzen des entsprechenden Alkalimetallsalzes in üblicher Weise hergestellt werden, ebenso Ammonium-, Phosphonium-, Sulfonium- und Sulfoxoniumsalze mittels Ammoniak, Phosphonium-, Sulfonium- oder Sulfoxoniumhydroxiden.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa , Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Des weiteren eignen sich die Benzylhydroxylamine I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermoglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, i die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder 0 Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Impragnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen etwa 0,001 bis 98 Gew.-%, vorzugsweise 0,01 bis 95 Gew-.%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die folgenden Formulierungsbeispiele verdeutlichen die Herstellung solcher Zubereitungen:
I. 20 Gewichtsteile der Verbindung Nr. It.10 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. 1.02 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. 1.03 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. 1.05 werden mit 3 Gewichtsteilen des Natriumsalzes der Diisobutylnaphthalin-a-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser enthält man eine Spritzbrühe, die 0,1 Gew.-% des Wirkstoffs enthält.
V. 3 Gewichtsteile des Wirkstoffs Nr. 1.04 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 1.16 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. I Gewichtsteil des Wirkstoffs Nr. 1.17 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. I Gewichtsteil des Wirkstoffs Nr. 1.18 wird in einer Mischung gelöst, die aus 80 Gewichtsteilen Cyclohexanon und 20 Gewichtsteilen Uniperol® EL (= nichtionischer Emulgator auf der Basis von ethoxyliertem Ricinusöl) besteht. Man erhält ein stabiles Emulsionskonzentrat.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die Benzylhydroxylamine I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder ) in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Herstellungsbeispiele

### Beispiel 1

### 3- (4-Chlor-3-methoxyaminomethyl-phenyl)-1-methyl-6-trifluormethyl-1,2,3,4-tetrahydro-pyrimidin-2,4-dion (Verbindung Nr. 1.01)

Zu einer Lösung von 3-(4-Chlor-3-methoxyiminomethyl-phenyl)-1-methyl-6-trifluormethyl-1,2,3,4-tetrahydro-pyrimidin-2,4-dion (3,6 g) in einer Mischung aus 30 ml Dichlormethan und 50 ml Trifluoressigsäure wurde Triethylsilan (1,9 ml) getropft. Nach 20 Std. Rühren bei Raumtemperatur destillierte man das Lösungsmittel ab. Der Rückstand wurde in 200 ml Dichlormethan aufgenommen, wonach die organische Phase viermal mit je 50 ml Wasser gewaschen, über Natriumsulfat getrocknet und schließlich eingeengt wurde. Nach Kristallisation mit Diisopropylether und Petrolether erhielt man die Titelverbindung (Smp. 83-85°C).

### Beispiel 2

### 3- (4-Chlor-3-ethoxyaminomethyl-phenyl)-1-methyl-6-trifluormethyl-1,2,3,4-tetrahydropyrimidin-2,4-dion (Verbindung Nr. 1.02)

Zu einer Lösung von 3-(4-Chlor-3-ethoxyiminomethyl-phenyl)-1-methyl-6-trifluormethyl-1,2,3,4-tetrahydropyrimidin-2,4-dion (3,8 g) in 40 ml Ethanol wurden bei 0°C nacheinander Boran-Pyridin-Komplex (3 ml) und 10 prozentige Salzsäure (30 ml) getropft. Innerhalb von 16 Std. wurden dann nochmals 12 ml Boran-Pyridin-Komplex zugegeben. Anschließend rührte man die Lösung 4 Std. bei Rückflußtemperatur, wonach das Lösungsmittel abdestilliert wurde. Den Rückstand nahm man in 200 ml Dichlormethan auf. Die organische Phase wurde zweimal mit je 50 ml Wasser gewaschen, dann über Natriumsulfat getrocknet und schließlich eingeengt. Man erhielt die Titelverbindung als Öl.

### Beispiel 3

### 3- (3-[Acetyl- (methoxyamino)-methyl]-4-chlor-phenyl)-1-methyl-6-trifluormethyl-1,2,3,4-tetrahydropyrimidin-2,4-dion (Verbindung Nr. 1.04)

Zu einer Suspension von Natriumhydrid (0,17 g in 50 ml Tetrahydrofuan) wurde 3-(4-Chlor-3-methoxyaminomethyl-phenyl)-1-methyl-6-trifluormethyl-1,2,3,4-tetrahydro-pyrimidin-2,4-dion (1,6 g in 30 ml Tetrahydrofuran) getropft. Nach einer Stunde wurde Acetylchlorid (0,4 g in 20 ml Tetrahydrofuran) zugegeben. Die Mischung wurde 20 Stunden bei Raumtemperatur gerührt und anschließend mit Wasser (100 ml) versetzt. Aus der wäßrigen Phase extrahierte man das Wertprodukt mit zweimal 100 ml Dichlormethan.

Die vereinigten organischen Phasen wurden dreimal mit Wasser gewaschen, über Natriumsulfat getrocknet und vom Lösungsmittel befreit. Nach Chromatographie an Kieselgel (Laufmittel: Dichlormethan/Ethylacetat = 9:1) und Kristallisation mit Petrolether erhielt man die Titelverbindung (Smp. 160-161°C).

### Beispiel 4

### 3- (3-[(Methoxy-methylamino)-methyl]-4-chlor-phenyl)-1-methyl-6-trifluormethyl-1,2,3,4-tetrahydropyrimidin-2,4-dion (Verbindung Nr. 1.06)

Zu einer Mischung von Methoxymethylamin (0,06 Mol), Kaliumcarbonat (0,012 Mol) und 100 ml Dimethylformamid wurden 3-(3-Brommethyl)-4-chlor-2-fluorphenyl)-1-methyl-6-trifluormethyl-1,2,3,4-tetrahydropyrimidin-2,4-dion (0,005 Mol) gegeben. Nach 5 Std. bei Raumtemperatur engte man die Reaktionslösung ein. Der Rückstand wurde mit 100 ml Methylenchlorid versetzt, wonach die organische Phase 3 mal mit je 30 ml Wasser gewaschen, dann über Natriumsulfat getrocknet und schließlich eingeengt wurde. Aus dem so erhaltenen Öl erhielt man durch Zugabe von Petrolether das gewünschte Wertprodukt.

### Beispiel 5

### 3-(3- [Ethoxycarbonyl-aminooxy-methyl]-4-chlor-6-fluor-phenyl)-1-amino-6-trifluormethyl-1,2,3,4-tetrahydropyrimidin-2,4-dion (Verbindung Nr. It.10)

Zu einer Lösung von 1,8 g 3-(3-Hydroxymethyl-4-chlor-6-fluorphenyl)-1-amino-6-trifluormethyl-1,2,3,4-tetrahydropyrimidin-2,4-dion in 100 ml Tetrahydrofuran wurden 0,9 g Carbonyldiimidazol gegeben. Nach 1 Std. Rühren versetzte man das Reaktionsgemisch mit 0,58 g N-Ethoxycarbonyl-hydroxylamin. Anschließend wurde die Mischung noch 14 Std. bei 20°C gerührt, wonach man das Lösungsmittel destillativ entfernte. Der Rückstand wurde in 100 ml Methylenchlorid aufgenommen. Die erhaltene organische Phase wurde mit Wasser gewaschen, über Natriumsulfat getrocknet und schließlich eingeengt. Die Reinigung des Rohprodukts erfolgte mittels Chromatographie an Kieselgel (Laufmittel: Methylenchlorid). Ausbeute: 0,2 g.

In der folgenden Tabelle 4 sind neben den in vorstehend beschriebenen Verbindungen noch weitere Benzylhydroxylamine I aufgeführt, die in analoger Weise hergestellt wurden oder herstellbar sind:

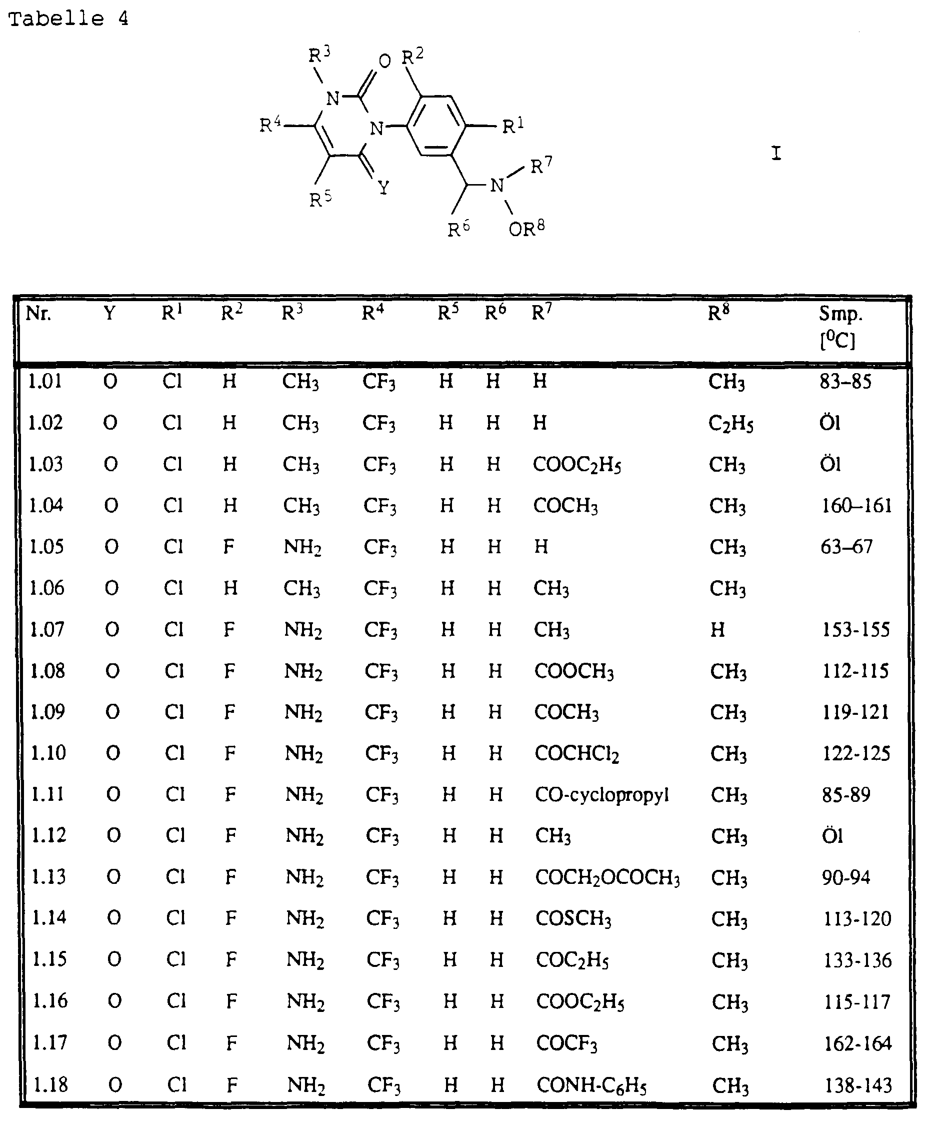

### Anwendungsbeispiele (herbizide Wirksamkeit)

Die herbizide Wirkung der Benzylhydroxylamine I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,0156, 0,0078, 0,0039 oder 0,0019 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name | |
|---|---|---|---|
| Abutilon theophrasti | Chinesischer Hanf | Velvet leaf | |
| Galium aparine | Klettenlabkraut | Catchweed bedstraw | |
| Sinapis alba | Weißer Senf | White mustard | |
| Solanum nigrum | Schwarzer Nachtschatten | Black nightshade | |
| Veronica subspecies | Ehrenpreisarten | Speedwell | |

Bei einer Aufwandmenge von 0,0156 oder 0,0078 kg/ha a.S. zeigte die Verbindung Nr. 1.03 im Nachauflaufverfahren eine sehr gute Wirkung gegen Abutilon theophrasti, Solanum nigrum und verschiedenen Veronica-Spezies.

Bei einer Aufwandmenge von 3,9 oder 1,9 g/ha a.S. zeigte die Verbindung Nr. It.10 im Nachauflaufverfahren eine sehr gute Wirkung gegen Galium aparine, Sinapis alba und Solanum nigrum.

### Anwendungsbeispiele (desikkative/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag/Nachttemperatur = 27/20°C).

Die jungen Baumwollpflanzen wurden tropfnaß mit wäßrigen Aufbereitungen der Wirkstoffe (unter Zusatz von 0,15 Gew.-% des Fettalkoholalkoxylats Plurafac LF 700, bezogen auf die Spritzbrühe) blattbehandelt. Die ausgebrachte Wassermenge betrug umgerechnet 1000 1/ha. Nach 13 Tagen wurde die Anzahl der abgeworfenen Blätter und der Grad der Entblätterung in % bestimmt.

Bei den unbehandelten Kontrollpflanzen trat kein Blattfall auf.

## Patentansprüche

1. Benzylhydroxylamine der Formel I 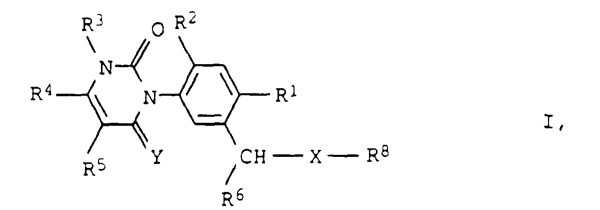 in der die Variablen folgende Bedeutung haben:
X -N(R⁷)-O-, das über Sauerstoff oder Stickstoff an R⁸ gebunden sein kann;
Y Sauerstoff oder Schwefel;
R¹ Halogen, Cyano, Nitro oder Trifluormethyl;
R² Wasserstoff oder Halogen;
R³ Wasserstoff, Amino oder Methyl;
R⁴ Wasserstoff, Halogen, C₁-C₆-Alkyl, C₁-C₆-Halogenalkyl, C₁-C₆-Alkylthio, C₁-C₆-Alkylsulfinyl oder C₁-C₆-Alkylsulfonyl;
R⁵ Wasserstoff, Halogen oder C₁-C₆-Alkyl;
R⁶ Wasserstoff, C₁-C₆-Alkyl, C₁-C₆-Halogenalkyl, C₃-C₆-Cycloalkyl oder C₂-C₆-Alkenyl;
R⁷ Wasserstoff, C₁-C₆-Alkyl, C₃-C₈-Cycloalkyl, C₁-C₆-Halogenalkyl, C₃-C₆-Alkenyl, C₃-C₆-Alkinyl, (C₁-C₆-Alkyl)carbonyl, (C₃-C₆-Alkenyl)carbonyl, (C₃-C₆-Alkinyl)carbonyl, (C₁-C₆-Alkoxy)carbonyl, (C₂-C₆-Alkenyloxy)carbonyl, (C₂-C₆-Alkinyloxy)carbonyl, (C₁-C₆-Alkylthio)carbonyl, C₁-C₆-Alkylsulfonyl, C₁-C₆-Alkylcarbamoyl, wobei jeder der letztgenannten 14 Reste gewünschtenfalls ein bis drei Substituenten tragen kann, jeweils ausgewählt aus der Gruppe bestehend aus
- Nitro, Cyano, Halogen, C₃-C₈-Cycloalkyl, Hydroxy, C₁-C₆-Alkoxy, C₃-C₈-Cycloalkoxy, C₃-C₆-Alkenyloxy, C₃-C₆-Alkinyloxy, C₁-C₆-Alkoxy-C₁-C₆-alkoxy, C₁-C₆-Alkylthio, (C₁-C₆-Alkyl)carbonyl, (C₁-C₆-Alkyl)carbonyloxy, C₁-C₆-Alkylsulfinyl, C₁-C₆-Alkylsulfonyl, C₁-C₆-Alkylidenaminoxy, C₁-C₆-Alkylcarbamoyl,
- der Phenyl-, Phenoxy- oder Phenylsulfonylgruppe, wobei die Phenylringe unsubstituiert sein oder ein bis drei Substituenten tragen können, jeweils ausgewählt aus der Gruppe bestehend aus Halogen, Nitro, Cyano, C₁-C₆-Alkyl, C₁-C₆-Alkoxy und C₁-C₆-Halogenalkyl,
- einer 3- bis 7-gliedrigen Heterocyclyl- oder Heterocyclyloxygruppe mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Halogen, Nitro, Cyano, C₁-C₆-Alkyl, C₁-C₆-Alkoxy, C₁-C₆-Halogenalkyl und (C₁-C₆-Alkyl)carbonyl,
- einer Gruppe -CO-Z¹R⁹, -OCO-Z¹R⁹ oder -N(R⁹)R¹⁰,
oder
R⁷ C₃-C₈-Cycloalkylcarbonyl, Phenylcarbonyl, Phenylsulfonyl oder Phenylcarbamoyl, wobei diese 4 Reste unsubstituiert sein oder ein bis drei Substituenten tragen können, jeweils ausgewählt aus der Gruppe bestehend aus Halogen, Nitro, Cyano, C₁-C₆-Alkyl, C₁-C₆-Alkoxy und C₁-C₆-Halogenalkyl;
R⁸ Wasserstoff, C₁-C₆-Alkyl, C₃-C₈-Cycloalkyl, C₁-C₆-Halogenalkyl, C₃-C₆-Alkenyl oder C₃-C₆-Alkinyl, wobei jeder der letztgenannten 5 Reste gewünschtenfalls ein bis drei Substituenten tragen kann, jeweils ausgewählt aus der Gruppe bestehend aus
- Nitro, Cyano, Halogen, C₃-C₈-Cycloalkyl, Hydroxy, C₁-C₆-Alkoxy, C₃-C₈-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfinyl, C₁-C₆-Alkylsulfonyl, C₁-C₆-Alkylidenaminoxy,
- der Phenyl-, Phenoxy- oder Phenylsulfonylgruppe, wobei die Phenylringe unsubstituiert sein oder ein bis drei Substituenten tragen können, jeweils ausgewählt aus der Gruppe bestehend aus Nitro, Cyano, Halogen, C₁-C₆-Alkyl, C₁-C₆-Alkoxy oder C₁-C₆-Halogenalkyl,
- einer 3- bis 7-gliedrigen Heterocyclyl- oder Heterocyclyloxygruppe mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Halogen, Nitro, Cyano, C₁-C₆-Alkyl, C₁-C₆-Alkoxy, C₁-C₆-Halogenalkyl oder (C₁-C₆-Alkyl)carbonyl,
- einer Gruppe -CO-Z²R¹¹, -OCO-^{Z2}R¹¹ oder -N(R¹¹)R¹²;
Z¹ eine chemische Bindung, Sauerstoff, Schwefel oder -N(R¹⁰)-;
Z² eine chemische Bindung, Sauerstoff, Schwefel oder -N(R¹²)-;
R⁹, R¹¹ unabhängig voneinander Wasserstoff, C₁-C₆-Alkyl, C₁-C₆-Halogenalkyl, C₃-C₈-Cycloalkyl, C₃-C₆-Alkenyl, C₃-C₆-Alkinyl, C₁-C₆-Alkoxy-C₁-C₆-alkyl, (C₁-C₆-Alkoxy)carbonyl-C₁-C₆-alkyl, Phenyl oder Phenyl-C₁-C₆-alkyl, wobei die Phenylgruppe und der Phenylring der Phenylalkylgruppe unsubstituiert sein oder einen bis drei Reste tragen können, jeweils ausgewählt aus der Gruppe bestehend aus Nitro, Cyano, Halogen, C₁-C₆-Alkyl, C₁-C₆-Halogenalkyl, C₁-C₆-Alkoxy oder (C₁-C₆-Alkyl)carbonyl
oder
Z¹ und R⁹ und/oder Z² und R¹¹ jeweils zusammen für einen über Stickstoff gebundenen 3- bis 7-gliedrigen Heterocyclus mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Nitro, Cyano, Halogen, C₁-C₆-Alkyl, C₁-C₆-Halogenalkyl und C₁-C₆-Alkoxy;
R¹⁰, R¹² unabhängig voneinander Wasserstoff, Hydroxy, C₁-C₆-Alkyl, C₃-C₈-Cycloalkyl oder C₁-C₆-Alkoxy,
sowie die landwirtschaftlich brauchbaren Salze derjenigen Verbindungen I, bei denen R³, R⁷ und/oder R⁸ Wasserstoff bedeuten.

2. Benzylhydroxylamine der Formel I gemäß Anspruch 1, wobei die Variablen folgende Bedeutung haben:
Y Sauerstoff;
R¹ Halogen oder Cyano;
R² Wasserstoff, Fluor oder Chlor;
R³ Amino oder Methyl;
R⁴ C₁-C₆-Alkyl, C₁-C₆-Halogenalkyl oder C₁-C₆-Alkylsulfonyl;
R⁵ Wasserstoff oder Halogen;
R⁶ Wasserstoff oder C₁-C₄-Alkyl;
R⁷ Wasserstoff, C₁-C₆-Alkyl, C₃-C₈-Cycloalkyl, C₁-C₆-Halogenalkyl, C₃-C₆-Alkenyl, C₃-C₆-Alkinyl, (C₁-C₆-Alkyl)carbonyl, (C₃-C₆-Alkenyl)carbonyl, (C₃-C₆-Alkinyl)carbonyl, (C₁-C₆-Alkoxy)carbonyl, (C₂-C₆-Alkenyloxy)carbonyl, (C₂-C₆-Alkinyloxy)carbonyl, (C₁-C₆-Alkylthio)carbonyl, C₁-C₆-Alkylcarbamoyl, wobei jeder der letztgenannten 13 Reste gewünschtenfalls einen oder zwei Substituenten tragen kann, jeweils ausgewählt aus der Gruppe bestehend aus
- Nitro, Cyano, Halogen, C₃-C₈-Cycloalkyl, Hydroxy, C₁-C₆-Alkoxy, C₃-C₈-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfinyl, C₁-C₆-Alkylsulfonyl, C₁-C₆-Alkylidenaminoxy,
- einer Gruppe -CO-Z¹R⁹, -OCO-Z¹R⁹ oder -N(R⁹)R¹⁰;
R⁸ Wasserstoff, C₁-C₆-Alkyl, C₃-C₈-Cycloalkyl, C₁-C₆-Halogenalkyl, C₃-C₆-Alkenyl oder C₃-C₆-Alkinyl, wobei jeder der letztgenannten 5 Reste gewünschtenfalls einen oder zwei Substituenten tragen kann, jeweils ausgewählt aus der Gruppe bestehend aus
- Nitro, Cyano, Halogen, C₃-C₈-Cycloalkyl, Hydroxy, C₁-C₆-Alkoxy, C₃-C₈-Cycloalkoxy, C₃-C₆-Alkenyloxy, C₃-C₆-Alkinyloxy, C₁-C₆-Alkoxy-C₁-C₆-alkoxy, C₁-C₆-Alkylthio, C₁-C₆-Alkylsulfinyl, C₁-C₆-Alkylsulfonyl, C₁-C₆-Alkylidenaminoxy,
- einer Gruppe -CO-Z²R¹¹, -OCO-Z²R¹¹ oder -N(R¹¹)R¹²;
Z¹ eine chemische Bindung, Sauerstoff, Schwefel oder -N(R¹⁰)-;
Z² eine chemische Bindung, Sauerstoff, Schwefel oder -N(R¹²)-;
R⁹, R¹¹ unabhängig voneinander Wasserstoff, C₁-C₆-Alkyl, C₃-C₈-Cycloalkyl, C₃-C₆-Alkenyl, C₃-C₆-Alkinyl, C₁-C₆-Alkoxy-C₁-C₆-alkyl oder (C₁-C₆-Alkoxy)carbonyl-C₁-C₆-alkyl;
oder
Z¹ und R⁹ und/oder Z² und R¹¹ jeweils zusammen für einen über Stickstoff gebundenen 3- bis 7-gliedrigen Heterocyclus mit ein bis drei Heteroatomen, ausgewählt aus der Gruppe bestehend aus zwei Sauerstoffatomen, zwei Schwefelatomen und 3 Stickstoffatomen, wobei der Heterocyclus gesättigt, partiell oder vollständig ungesättigt oder aromatisch sein und gewünschtenfalls ein bis drei Substituenten tragen kann, jeweils ausgewählt aus der Gruppe bestehend aus Nitro, Cyano, Halogen, C₁-C₆-Alkyl, C₁-C₆-Halogenalkyl und C₁-C₆-Alkoxy;
R¹⁰, R¹² unabhängig voneinander Wasserstoff oder C₁-C₆-Alkyl.

3. Verwendung der Benzylhydroxylamine der Formel I und der landwirtschaftlich brauchbaren Salze von I, gemäß Anspruch 1, als Herbizide oder zur Desikkation und/oder Defoliation von Pflanzen.

4. Herbizides Mittel, enthaltend eine herbizid wirksame Menge mindestens eines Benzylhydroxylamins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

5. Mittel zur Desikkation und/oder Defoliation von Pflanzen, enthaltend eine desikkant und/oder defoliant wirksame Menge mindestens eines Benzylhydroxylamins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

6. Verfahren zur Herstellung von herbizid wirksamen Mitteln, dadurch gekennzeichnet, daß man eine herbizid wirksame Menge mindestens eines Benzylhydroxylamins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

7. Verfahren zur Herstellung von desikkant und/oder defoliant wirksamen Mitteln, dadurch gekennzeichnet, daß man eine desikkant und/oder defoliant wirksame Menge mindestens eines Benzylhydroxylamins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

8. Verfahren zur Bekämpfung von unerwünschtem Pflanzenwuchs, dadurch gekennzeichnet, daß man eine herbizid wirksame Menge mindestens eines Benzylhydroxylamins der Formel I oder eines landwirtschaftlich brauchbaren Salzes von I, gemäß Anspruch 1, auf Pflanzen, deren Lebensraum oder auf Saatgut einwirken läßt.

9. Verfahren zur Desiccation und/oder Defoliation von Pflanzen, dadurch gekennzeichnet, daß man eine desikkant und/oder defoliant wirksame Menge mindestens eines Benzylhydroxylamins der Formel I oder eines landwirtschaftlich brauchbaren Salzes von I, gemäß Anspruch 1, auf Pflanzen einwirken läßt.

10. Enaminester der Formel IV 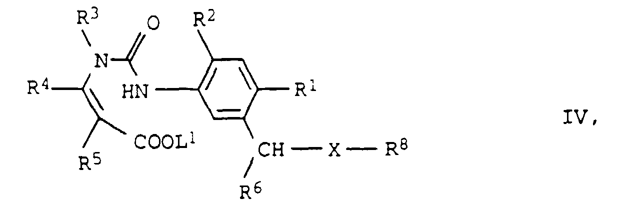 in der L¹ für C₁-C₆-alkyl oder Phenyl steht und die Substituenten R¹ bis R⁸ die in Anspruch 1 angegebenen Bedeutungen haben.

11. Enamincarboxylate der Formel V 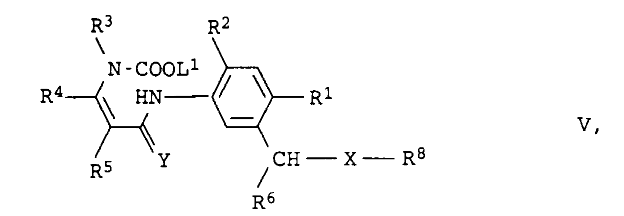 in der L¹ für C₁-C₆-Alkyl oder Phenyl steht und die Substituenten R¹ bis R^{8,} X und Y die in Anspruch 1 angegebenen Bedeutungen haben.

12. Phenylharnstoffderivate der Formel XIII 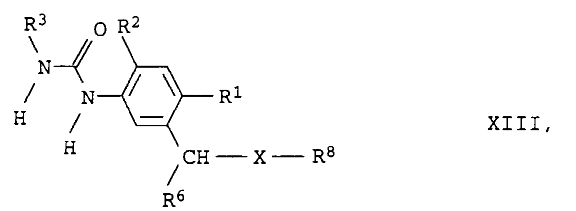 in der die Substituenten R¹ bis R³ und R⁶ bis R⁸ die in Anspruch 1 angegebenen Bedeutungen haben.

13. Phenylisocyanate der Formel XVI 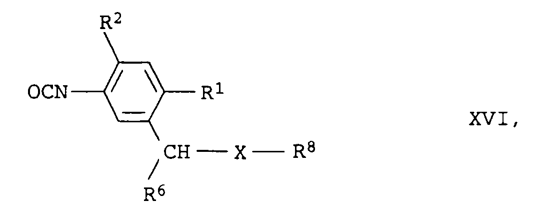 in der die Substituenten R¹, R² und R⁶ bis R⁸ die in Anspruch 1 angegebenen Bedeutungen haben.

14. Anilinderivate der Formel XVII 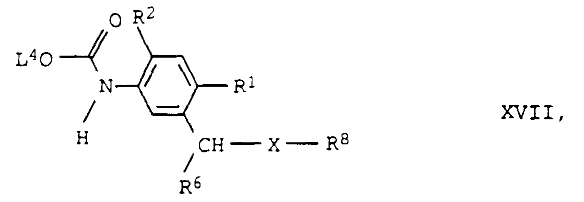 in der L⁴ für C₁-C₄-Alkyl oder Phenyl steht und die Substituenten R¹, R² und R⁶ bis R⁸ die in Anspruch 1 angegebenen Bedeutungen haben.

15. Anilin-Verbindungen der Formel XIX 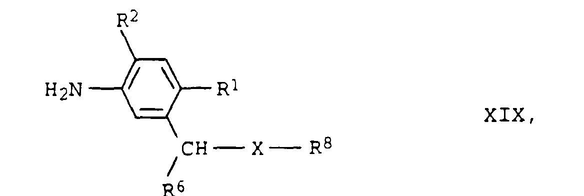 in der die Substituenten R¹, R² und R⁶ bis R⁸ die in Anspruch 1 angegebenen Bedeutungen haben.

16. Nitro-Verbindungen der Formel XXIII 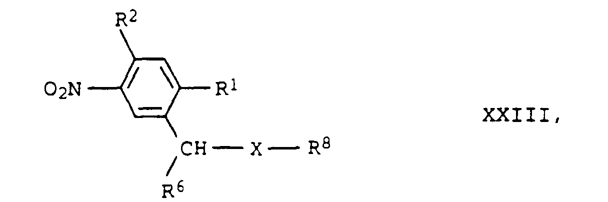 in der die Substituenten R¹, R² und R⁶ bis R⁸ die in Anspruch 1 angegebenen Bedeutungen haben.

## Claims

1. A benzylhydroxylamine of the formula I 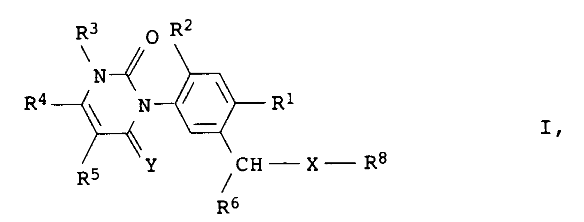 in which the variables have the following meanings:
X is -N(R⁷)-O- which can be bonded to R⁸ via oxygen or nitrogen;
Y is oxygen or sulfur;
R¹ is halogen, cyano, nitro or trifluoromethyl;
R² is hydrogen or halogen;
R³ is hydrogen, amino or methyl;
R⁴ is hydrogen, halogen, C₁-C₆-alkyl, C₁-C₆-haloalkyl, C₁-C₆-alkylthio, C₁-C₆-alkylsulfinyl or C₁-C₆-alkylsulfonyl;
R⁵ is hydrogen, halogen or C₁-C₆-alkyl;
R⁶ is hydrogen, C₁-C₆-alkyl, C₁-C₆-haloalkyl, C₃-C₆-cycloalkyl or C₂-C₆-alkenyl;
R⁷ is hydrogen, C₁-C₆-alkyl, C₃-C₈-cycloalkyl, C₁-C₆-haloalkyl, C₃-C₆-alkenyl, C₃-C₆-alkynyl, (C₁-C₆-alkyl)carbonyl, (C₃-C₆-alkenyl)carbonyl, (C₃-C₆-alkynyl)carbonyl, (C₁-C₆-alkoxy)carbonyl, (C₂-C₆-alkenyloxy)carbonyl, (C₂-C₆-alkynyloxy)carbonyl, (C₁-C₆-alkylthio)carbonyl, C₁-C₆-alkylsulfonyl, C₁-C₆-alkylcarbamoyl, it optionally being possible for each of the last-mentioned 14 radicals to have attached to it one to three substituents in each case selected from the group consisting of
- nitro, cyano, halogen, C₃-C₈-cycloalkyl, hydroxyl, C₁-C₆-alkoxy, C₃-C₈-cycloalkoxy, C₃-C₆-alkenyloxy, C₃-C₆-alkynyloxy, C₁-C₆-alkoxy-C₁-C₆-alkoxy, C₁-C₆-alkylthio, (C₁-C₆-alkyl)carbonyl, (C₁-C₆-alkyl)carbonyloxy, C₁-C₆-alkylsulfinyl, C₁-C₆-alkylsulfonyl, C₁-C₆-alkylideneaminoxy, C₁-C₆-alkylcarbamoyl,
- the phenyl, phenoxy or phenylsulfonyl group, it being possible for the phenyl rings to be unsubstituted or to have attached to them one to three substituents, in each case selected from the group consisting of halogen, nitro, cyano, C₁-C₆-alkyl, C₁-C₆-alkoxy and C₁-C₆-haloalkyl,
- a 3- to 7-membered heterocyclyl or heterocyclyloxy group having one to three hetero atoms, selected from the group consisting of two oxygen atoms, two sulfur atoms and 3 nitrogen atoms, it being possible for the heterocycle to be saturated, partially or fully unsaturated or aromatic, and, if desired, to have attached to it one to three substituents, in each case selected from the group consisting of halogen, nitro, cyano, C₁-C₆-alkyl, C₁-C₆-alkoxy, C₁-C₆-haloalkyl and (C₁-C₆-alkyl)carbonyl,
- a group -CO-Z¹R⁹, -OCO-Z¹R⁹ or -N(R⁹)R¹⁰,
or
R⁷ is C₃-C₈-cycloalkylcarbonyl, phenylcarbonyl, phenylsulfonyl or phenylcarbamoyl, it being possible for these 4 radicals to be unsubstituted or to have attached to them one to three substituents, in each case selected from the group consisting of halogen, nitro, cyano, C₁-C₆-alkyl, C₁-C₆-alkoxy and C₁-C₆-haloalkyl;
R⁸ is hydrogen, C₁-C₆-alkyl, C₃-C₈-cycloalkyl, C₁-C₆-haloalkyl, C₃-C₆-alkenyl or C₃-C₆-alkynyl, it being possible, if desired, for each of the last-mentioned 5 radicals to have attached to it one to three substituents, in each case selected from the group consisting of
- nitro, cyano, halogen, C₃-C₈-cycloalkyl, hydroxyl, C₁-C₆-alkoxy, C₃-C₈-cycloalkoxy, C₃-C₆-alkenyloxy, C₃-C₆-alkynyloxy, C₁-C₆-alkoxy-C₁-C₆-alkoxy, C₁-C₆-alkylthio, C₁-C₆-alkylsulfinyl, C₁-C₆-alkylsulfonyl, C₁-C₆-alkylideneaminoxy,
- the phenyl, phenoxy or phenylsulfonyl group, it being possible for the phenyl rings to be unsubstituted or to have attached to them one to three substituents, in each case selected from the group consisting of nitro, cyano, halogen, C₁-C₆-alkyl, C₁-C₆-alkoxy and C₁-C₆-haloalkyl,
- a 3- to 7-membered heterocyclyl or heterocyclyloxy group having one to three hetero atoms, selected from the group consisting of two oxygen atoms, two sulfur atoms and 3 nitrogen atoms, it being possible for the heterocycle to be saturated, partially or fully unsaturated or aromatic, and, if desired, to have attached to it one to three substituents, in each case selected from the group consisting of halogen, nitro, cyano, C₁-C₆-alkyl, C₁-C₆-alkoxy, C₁-C₆-haloalkyl and (C₁-C₆-alkyl)carbonyl,
- a group -CO-Z²R¹¹, -OCO-Z²R¹¹ or -N(R¹¹)R¹²;
Z¹ is a chemical bond, oxygen, sulfur or -N(R¹⁰)-;
Z² is a chemical bond, oxygen, sulfur or -N(R¹²)-;
R⁹, R¹¹ independently of one another are hydrogen, C₁-C₆-alkyl, C₁-C₆-haloalkyl, C₃-C₈-cycloalkyl, C₃-C₆-alkenyl, C₃-C₆-alkynyl, C₁-C₆-alkoxy-C₁-C₆-alkyl, (C₁-C₆-alkoxy)carbonyl-C₁-C₆-alkyl, phenyl or phenyl-C₁-C₆-alkyl, it being possible for the phenyl group and the phenyl ring of the phenylalkyl group to be unsubstituted or to have attached to them one to three radicals, in each case selected from the group consisting of nitro, cyano, halogen, C₁-C₆-alkyl, C₁-C₆-haloalkyl, C₁-C₆-alkoxy or (C₁-C₆-alkyl)carbonyl,
or
Z¹ and R⁹ and/or Z² and R¹¹ in each case together are a 3- to 7-membered heterocycle bonded via nitrogen and having one to three hetero atoms selected from the group consisting of two oxygen atoms, two sulfur atoms and 3 nitrogen atoms, it being possible for the heterocycle to be saturated, partially or fully unsaturated or aromatic and, if desired, to have attached to it one to three substituents in each case selected from the group consisting of nitro, cyano, halogen, C₁-C₆-alkyl, C₁-C₆-haloalkyl and C₁-C₆-alkoxy;
R¹⁰, R¹² independently of one another are hydrogen, hydroxyl, C₁-C₆-alkyl, C₃-C₈-cycloalkyl or C₁-C₆-alkoxy,
or an agriculturally useful salt of a compound I where R³, R⁷ and/or R⁸ is hydrogen.

2. A benzylhydroxylamine of the formula I as claimed in claim 1, where the variables have the following meanings:
Y is oxygen;
R¹ is halogen or cyano;
R² is hydrogen, fluorine or chlorine;
R³ is amino or methyl;
R⁴ is C₁-C₆-alkyl, C₁-C₆-haloalkyl or C₁-C₆-alkylsulfonyl;
R⁵ is hydrogen or halogen;
R⁶ is hydrogen or C₁-C₄-alkyl;
R⁷ is hydrogen, C₁-C₆-alkyl, C₃-C₈-cycloalkyl, C₁-C₆-halo-alkyl, C₃-C₆-alkenyl, C₃-C₆-alkynyl, (C₁-C₆-alkyl)-carbonyl, (C₃-C₆-alkenyl)carbonyl, (C₃-C₆-alkynyl)carbonyl, (C₁-C₆-alkoxy)carbonyl, (C₂-C₆-alkenyloxy)carbonyl, (C₂-C₆-alkynyloxy)carbonyl, (C₁-C₆-alkylthio)carbonyl, C₁-C₆-alkylcarbamoyl, it being possible, if desired, for each of the last-mentioned 13 radicals to have attached to it one or two substituents, in each case selected from the group consisting of
- nitro, cyano, halogen, C₃-C₈-cycloalkyl, hydroxyl, C₁-C₆-alkoxy, C₃-C₈-cycloalkoxy, C₃-C₆-alkenyloxy, C₃-C₆-alkynyloxy, C₁-C₆-alkoxy-C₁-C6-alkoxy, C₁-C₆-alkylthio, C₁-C₆-alkylsulfinyl, C₁-C₆-alkylsulfonyl, C₁-C₆-alkylideneaminoxy, and
- a group -CO-Z¹R⁹, -OCO-Z¹R⁹ or -N(R⁹)R¹⁰;
R⁸ is hydrogen, C₁-C₆-alkyl, C₃-C₈-cycloalkyl, C₁-C₆-halo-alkyl, C₃-C₆-alkenyl or C₃-C₆-alkynyl, it being possible, if desired, for each of the 5 last-mentioned radicals to have attached to it one or two substituents, in each case selected from the group consisting of
- nitro, cyano, halogen, C₃-C₈-cycloalkyl, hydroxyl, C₁-C₆-alkoxy, C₃-C₈-cycloalkoxy, C₃-C₆-alkenyloxy, C₃-C₆-alkynyloxy, C₁-C₆-alkoxy-C₁-C₆-alkoxy, C₁-C₆-alkylthio, C₁-C₆-alkylsulfinyl, C₁-C₆-alkylsulfonyl, C₁-C₆-alkylideneaminoxy, and
- a group -CO-Z²R¹¹, -OCO-Z²R¹¹ or -N(R¹¹)R¹²;
Z1 is a chemical bond, oxygen, sulfur or -N(R¹⁰)-;
Z² is a chemical bond, oxygen, sulfur or -N(R¹²)-;
R⁹, R¹¹ independently of one another are hydrogen, C₁-C₆-alkyl, C₃-C₈-cycloalkyl, C₃-C₆-alkenyl, C₃-C₆-alkynyl, C₁-C₆-alkoxy-C₁-C₆-alkyl or (C₁-C₆-alkoxy)carbonyl-C₁-C₆-alkyl;
or
Z¹ and R⁹ and/or Z² and R¹¹ in each case together form a 3- to 7-membered heterocycle bonded via nitrogen and having one to three hetero atoms selected from the group consisting of two oxygen atoms, two sulfur atoms and 3 nitrogen atoms, it being possible for the heterocycle to be saturated, partially or fully unsaturated or aromatic and, if desired, to have attached to it one to three substituents, in each case selected from the group consisting of nitro, cyano, halogen, C₁-C₆-alkyl, C₁-C₆-haloalkyl and C₁-C₆-alkoxy;
R¹⁰, R¹² independently of one another are hydrogen or C₁-C₆-alkyl.

3. The use of a benzylhydroxylamine of the formula I and of an agriculturally useful salt of I, as claimed in claim 1, as herbicides or for the desiccation and/or defoliation of plants.

4. A herbicidal composition comprising a herbicidally active amount of at least one benzylhydroxylamine of the formula I or at least one agriculturally useful salt of I as claimed in claim 1 and at least one inert liquid and/or solid carrier and, if desired, at least one surfactant.

5. A composition for the desiccation and/or defoliation of plants, comprising an amount of at least one benzylhydroxylamine of the formula I or at least one agriculturally useful salt of I as claimed in claim 1 which has a desiccant and/or defoliant action and at least one inert liquid and/or solid carrier and, if desired, at least one surfactant.

6. A process for the preparation of herbicidally active compositions, which comprises mixing a herbicidally active amount of at least one benzylhydroxylamine of the formula I or at least one agriculturally useful salt of I as claimed in claim 1 and at least one inert liquid and/or solid carrier and, if desired, at least one surfactant.

7. A process for the preparation of compositions having a desiccant and/or defoliant action, which comprises mixing an amount of at least one benzylhydroxylamine of the formula I or at least one agriculturally useful salt of I as claimed in claim 1 which has a desiccant and/or defoliant action and at least one inert liquid and/or solid carrier and, if desired, at least one surfactant.

8. A method of controlling undesirable vegetation, which comprises allowing a herbicidally active amount of at least one benzylhydroxylamine of the formula I or at least one agriculturally useful salt of I as claimed in claim 1 to act on plants, their environment or on seed.

9. A method for the desiccation and/or defoliation of plants, which comprises allowing an amount of at least one benzylhydroxylamine of the formula I or at least one agriculturally useful salt of I as claimed in claim 1 which has a desiccant and/or defoliant action to act on plants.

10. An enamine ester of the formula IV 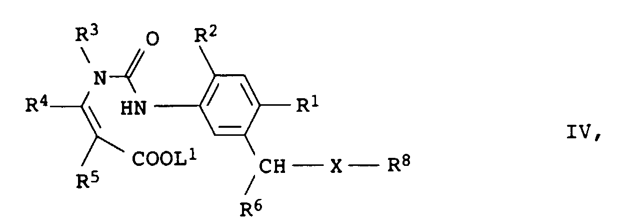 where L¹ is C₁-C₆-alkyl or phenyl and the substituents R¹ to R⁸ have the meanings given in claim 1.

11. An enamine carboxylate of the formula V 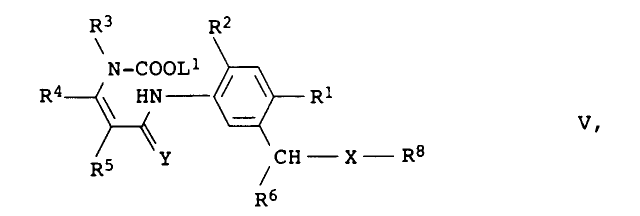 where L¹ is C₁-C₆-alkyl or phenyl and the substituents R¹ to R⁸, X and Y have the meanings given in claim 1.

12. A phenylurea derivative of the formula XIII 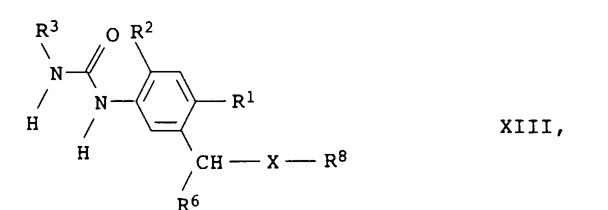 where the substituents R¹ to R³ and R⁶ to R⁸ have the meanings given in claim 1.

13. A phenylisocyanate of the formula XVI 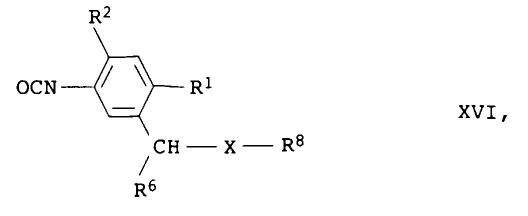 where the substituents R¹, R² and R⁶ to R⁸ have the meanings given in claim 1.

14. An aniline derivative of the formula XVII 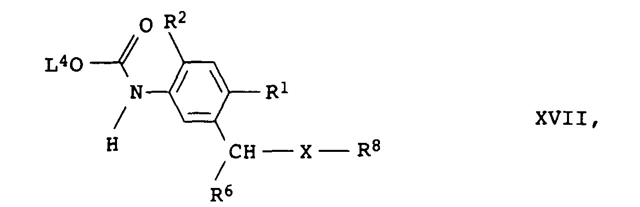 where L⁴ is C₁-C₄-alkyl or phenyl and the substituents R¹, R² and R⁶ to R⁸ have the meanings given in claim 1.

15. An aniline compound of the formula XIX 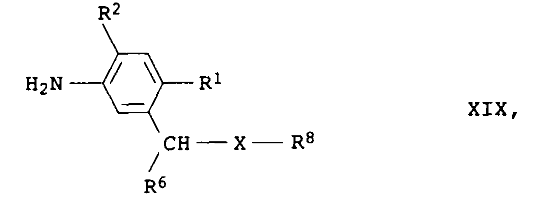 where the substituents R¹, R² and R⁶ to R⁸ have the meanings given in claim 1.

16. A nitro compound of the formula XXIII 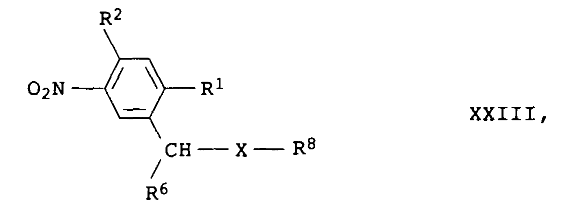 where the substituents R¹, R² and R⁶ to R⁸ have the meanings given in claim 1.

## Revendications

1. Benzylhydroxylamines de formule I 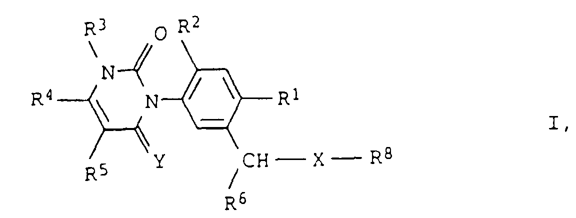 dans laquelle les symboles ont les significations suivantes :
X représente -N(R⁷)-O- qui peut être relié à R⁸ par l'intermédiaire de l'oxygène ou de l'azote ;
Y représente l'oxygène ou le soufre ;
R¹ représente un halogène, un groupe cyano, nitro ou trifluorométhyle ;
R² représente l'hydrogène ou un halogène ;
R³ représente l'hydrogène, un groupe amino ou méthyle ;
R⁴ représente l'hydrogène, un halogène, un groupe alkyle en C1-C6, halogénoalkyle en C1-C6, alkylthio en C1-C6, alkylsulfinyle en C1-C6 ou alkylsulfonyle en C1-C6 ;
R⁵ représente l'hydrogène, un halogène ou un groupe alkyle en C1-C6 ;
R⁶ représente l'hydrogène, un groupe alkyle en C1-C6, halogénoalkyle en C1-C6, cycloalkyle en C3-C6 ou alcényle en C2-C6 ;
R⁷ représente l'hydrogène, un groupe alkyle en C1-C6, cycloalkyle en C3-C8, halogénoalkyle en C1-C6, alcényle en C3-C6, alcynyle en C3-C6, (alkyle en Cl-C6)carbonyle, (alcényle en C3-C6)carbonyle, (alcynyle en C3-C6)carbonyle, (alcoxy en C1-C6)carbonyle, (alcényloxy en C2-C6)carbonyle, (alcynyloxy en C2-C6)carbonyle, (alkylthio en C1-C6)carbonyle, alkylsulfonyle en C1-C6, (alkyle en Cl-C6)carbamoyle, chacun des quatorze radicaux mentionnés en dernier pouvant le cas échéant porter un à trois substituants choisis parmi les suivants :
- nitro, cyano, halogéno, cycloalkyle en C3-C8, hydroxy, alcoxy en C1-C6, cycloalcoxy en C3-C8, alcényloxy en C3-C6, alcynyloxy en C3-C6, (alcoxy en C1-C6)alcoxy en C1-C6, alkylthio en C1-C6, (alkyle en C1-C6)carbonyle, (alkyle en Cl-C6)carbonyloxy, alkylsulfinyle en C1-C6, alkylsulfonyle en C1-C6, (alkylidène en C1-C6)aminoxy, (alkyle en C1-C6)carbamoyle,
- un groupe phényle, phénoxy ou phénylsulfonyle dans lesquels les cycles phényle peuvent être non substitués ou peuvent porter un à trois substituants choisis parmi les halogènes, les groupes nitro, cyano, alkyle en C1-C6, alcoxy en C1-C6 et halogénoalkyle en C1-C6,
- un groupe hétérocyclyle ou hétérocyclyloxy de trois à sept chaînons contenant un à trois hétéroatomes choisis dans le groupe consistant en deux atomes d'oxygène, deux atomes de soufre en trois atomes d'azote, l'hétérocycle pouvant être saturé, partiellement ou totalement insaturé ou aromatique et pouvant porter le cas échéant un à trois substituants choisis parmi les halogènes, les groupes nitro, cyano, alkyle en C1-C6, alcoxy en C1-C6, halogénoalkyle en C1-C6 et (alkyle en C1-C6)carbonyle,
- un groupe -CO-Z¹R⁹, -OCO-Z¹R⁹ ou -N(R⁹)R¹⁰,
ou bien
R⁷ représente un groupe (cycloalkyle en C3-C8)carbonyle, phénylcarbonyle, phénylsulfonyle ou phénylcarbamoyle, ces quatre radicaux pouvant être non substitués ou pouvant porter un à trois substituants choisis parmi les halogènes, les groupes nitro, cyano, alkyle en C1-C6, alcoxy en C1-C6 et halogénoalkyle en C1-C6 ;
R⁸ représente l'hydrogène, un groupe alkyle en C1-C6, cycloalkyle en C3-C8, halogénoalkyle en C1-C6, alcényle en C3-C6 ou alcynyle en C3-C6, chacun des cinq radicaux mentionnés en dernier pouvant porter le cas échéant un à trois substituants choisis parmi les suivants :
- nitro, cyano, halogéno, cycloalkyle en C3-C8, hydroxy, alcoxy en C1-C6, cycloalcoxy en C3-C8, alcényloxy en C3-C6, alcynyloxy en C3-C6, (alcoxy en Cl-C6)alcoxy en C1-C6, alkylthio en C1-C6, alkylsulfinyle en C1-C6, alkylsulfonyle en C1-C6, (alkylidène en C1-C6)aminoxy,
- un groupe phényle, phénoxy ou phénylsulfonyle dans lesquels les cycles phényle peuvent être non substitués ou peuvent porter un à trois substituants choisis parmi les groupes nitro, cyano, les halogènes, les groupes alkyle en C1-C6, alcoxy en C1-C6 ou hélogénoalkyle en C1-C6,
- un groupe hétérocyclyle ou hétérocyclyloxy de trois à sept chaînons contenant un à trois hétéroatomes choisis dans le groupe consistant en deux atomes d'oxygène, deux atomes de soufre et trois atomes d'azote, l'hétérocycle pouvant être saturé, partiellement ou totalement insaturé ou aromatique et pouvant porter le cas échéant un à trois substituants choisis parmi les halogènes, les groupes nitro, cyano, alkyle en C1-C6, alcoxy en C1-C6, halogénoalkyle en C1-C6 ou (alkyle en C1-C6)carbonyle,
- un groupe -CO-Z²R¹¹, -OCO-Z²R¹¹ ou -N(R¹¹)R¹² ;
Z¹ représente une liaison chimique, l'oxygène, le soufre ou -N(R¹⁰)- ;
Z² représente une liaison chimique, l'oxygène, le soufre ou -N(R¹²)- ;
R⁹ et R¹¹ représentent chacun, indépendamment l'un de l'autre, l'hydrogène, un groupe alkyle en C1-C6, halogénoalkyle en C1-C6, cycloalkyle en C3-C8, alcényle en C3-C6, alcynyle en C3-C6, (alcoxy en C1-C6)alkyle en C1-C6, (alcoxy en Cl-C6)carbonyl-alkyle en C1-C6, phényle ou phényl-alkyle en C1-C6, le groupe phényle et le cycle phényle du groupe phénylalkyle pouvant être non substitués ou pouvant porter un à trois substituants choisis parmi les groupes nitro, cyano, les halogènes, les groupes alkyle en C1-C6, halogénoalkyle en C1-C6, alcoxy en C1-C6 ou (alkyle en C1-C6)carbonyle
ou bien
Z¹ et R⁹ et/ou Z² et R¹¹ peuvent former ensemble, respectivement, un hétérocycle de trois à sept chaînons relié par l'intermédiaire de l'azote, contenant un à trois hétéroatomes choisis dans le groupe consistant en deux atomes d'oxygène, deux atomes de soufre et trois atomes d'azote, l'hétérocycle pouvant être saturé, partiellement ou totalement insaturé ou aromatique et pouvant porter le cas échéant un à trois substituants choisis parmi les groupes nitro, cyano, les halogènes, les groupes alkyle en C1-C6, halogénoalkyle en C1-C6 et alcoxy en C1-C6 ;
R¹⁰, R¹² représentent chacun, indépendamment l'un de l'autre, l'hydrogène, un groupe hydroxy, alkyle en C1-C6, cycloalkyle en C3-C8 ou alcoxy en C1-C6,
et les sels, convenant pour les applications agricoles, des composés I pour lesquels R³, R⁷ et/ou R⁸ représentent l'hydrogène.

2. Benzylhydroxylamines de formule I de la revendication 1, dans laquelle les symboles ont les significations suivantes :
Y l'oxygène ;
R¹ un halogène ou un groupe cyano ;
R² l'hydrogène, le fluor ou le chlore ;
R³ un groupe amino ou méthyle ;
R⁴ un groupe alkyle en C1-C6, halogénoalkyle en C1-C6 ou alkylsulfonyle en C1-C6 ;
R⁵ l'hydrogène ou un halogène ;
R⁶ l'hydrogène ou un groupe alkyle en C1-C4 ;
R⁷ l'hydrogène, un groupe alkyle en C1-C6, cycloalkyle en C3-C8, halogénoalkyle en C1-C6, alcényle en C3-C6, alcynyle en C3-C6, (alkyle en Cl-C6)carbonyle, (alcényle en C3-C6)carbonyle, (alcynyle en C3-C6)carbonyle, (alcoxy en Cl-C6)carbonyle, (alcényloxy en C2-C6)carbonyle, (alcynyloxy en C2-C6)carbonyle, (alkylthio en C1-C6)carbonyle, alkyle en Cl-C6)carbamoyle, chacun des treize radicaux mentionnés en dernier pouvant le cas échéant porter un ou deux substituants choisis parmi les suivants :
- nitro, cyano, halogéno, cycloalkyle en C3-C8, hydroxy, alcoxy en C1-C6, cycloalcoxy en C3-C8, alcényloxy en C3-C6, alcynyloxy en C3-C6, (alcoxy en Cl-C6)alcoxy en C1-C6, alkylthio en C1-C6, alkylsulfinyle en C1-C6, alkylsulfonyle en C1-C6, (alkylidène en Cl-C6)aminoxy,
- un groupe -CO-Z¹R⁹, -OCO(Z¹R⁹ ou -N(R9)R¹⁰ ;
R⁸ : l'hydrogène, un groupe alkyle en C1-C6, cycloalkyle en C3-C8, halogénoalkyle en C1-C6, alcényle en C3-C6 ou alcynyle en C3-C6, chacun des cinq radicaux mentionnés en dernier pouvant porter le cas échéant un ou deux substituants choisis parmi les suivants :
- nitro, cyano, halogéno, cycloalkyle en C3-C8, hydroxy, alcoxy en C1-C6, cycloalcoxy en C3-C8, alcényloxy en C3-C6, alcynyloxy en C3-C6, (alcoxy en Cl-C6)alcoxy en C1-C6, alkylthio en C1-C6, alkylsulfinyle en C1-C6, alkylsulfonyle en C1-C6, (alkylidène en C1-C6)aminoxy,
- un groupe -CO-Z²R¹¹, -OCO-Z²R¹¹ ou -N(R¹¹)R¹² ;
Z¹ : une liaison chimique, l'oxygène, le soufre ou -N(R¹⁰)- ;
Z² : une liaison chimique, l'oxygène, le soufre ou -N(R¹²)- ;
R⁹ et R¹¹, indépendamment l'un de l'autre : l'hydrogène, un groupe alkyle en C1-C6, cycloalkyle en C3-C8, alcényle en C3-C6, alcynyle en C3-C6, (alcoxy en C1-C6)alkyle en C1-C6 ou (alcoxy en C1-C6)carbonyl-alkyle en C1-C6 ;
ou bien
Z¹ et R⁹ et/ou Z² et R¹¹ forment ensemble, respectivement, un hétérocycle de trois à sept chaînons relié par l'intermédiaire de l'azote et contenant un à trois hétéroatomes choisis dans le groupe consistant en deux atomes d'oxygène, deux atomes de soufre et trois atomes d'azote, l'hétérocycle pouvant être saturé, partiellement ou totalement insaturé ou aromatique et pouvant porter le cas échéant un à trois substituants choisis parmi les groupes nitro, cyano, les halogènes, les groupes alkyle en C1-C6, halogénoalkyle en C1-C6 et alcoxy en C1-C6 ;
R¹⁰ et R¹² représentent chacun, indépendamment l'un de l'autre, l'hydrogène ou un groupe alkyle en C1-C6.

3. Utilisation des benzylhydroxylamines de formule I et des sels de I convenant pour les applications agricoles, selon la revendication 1, en tant qu'herbicides ou pour la dessiccation et/ou la défoliation de végétaux.

4. Produit herbicide contenant une quantité herbicide efficace d'au moins une benzylhydroxylamine de formule I ou d'un sel de I apte aux applications agricoles selon la revendication 1 et au moins un véhicule liquide et/ou solide inerte avec, le cas échéant, au moins une substance tensio-active.

5. Produit pour la dessiccation et/ou la défoliation de végétaux, contenant une quantité dessiccante et/ou défoliante efficace d'au moins une benzylhydroxylamine de formule I ou d'un sel I apte aux applications agricoles, selon la revendication 1, et au moins un véhicule liquide et/ou solide inerte avec, le cas échéant, au moins une substance tensio-active.

6. Procédé pour préparer des produits à activité herbicide, caractérisé par le fait que l'on mélange une quantité herbicide efficace d'au moins une benzylhydroxylamine de formule I ou d'un sel de I apte aux applications agricoles, selon la revendication 1, et au moins un véhicule liquide et/ou solide inerte, avec le cas échéant au moins une substance tensio-active.

7. Procédé pour la préparation de produits à activité dessiccante et/ou défoliante, caractérisé par le fait que l'on mélange une quantité dessiccante et/ou défoliante efficace d'au moins une benzylhydroxylamine de formule I ou d'un sel de I apte aux applications agricoles selon la revendication 1, et au moins un véhicule liquide et/ou solide inerte, avec, le cas échéant, au moins une substance tensio-active.

8. Procédé pour combattre les croissances de végétaux indésirables, caractérisé par le fait que l'on fait agir une quantité herbicide efficace d'au moins une benzylhydroxylamine de formule I ou d'un sel de I apte aux applications agricoles selon la revendication 1, sur les végétaux, leur habitat ou leurs semences.

9. Procédé pour la dessiccation et/ou la défoliation de végétaux, caractérisé par le fait que l'on fait agir une quantité dessiccante et/ou défoliante efficace d'au moins une benzylhydroxylamine de formule I ou d'un sel de I apte aux applications agricoles, selon la revendication 1, sur les végétaux.

10. Enamine-esters de formule IV 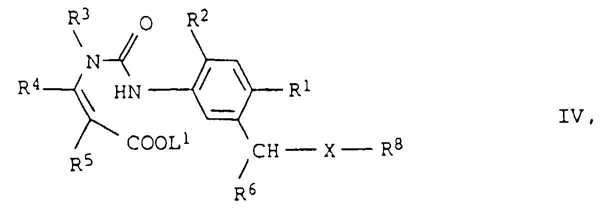 dans laquelle L¹ représente un groupe alkyle en C1-C6 ou phényle et les symboles R¹ à R⁸ ont les significations indiquées dans la revendication 1.

11. Enamine-carboxylates de formule V 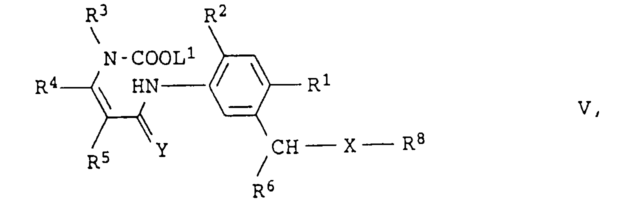 dans laquelle L¹ représente un groupe alkyle en C1-C6 ou phényle et les symboles R¹ à R⁸, X et Y ont les significations indiquées dans la revendication 1.

12. Dérivés de la phénylurée répondant à la formule XIII 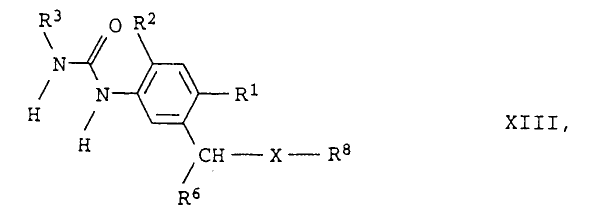 dans laquelle les symboles R¹ à R³ et R⁶ à R⁸ ont les significations indiquées dans la revendication 1.

13. Phénylisocyanates de formule XVI 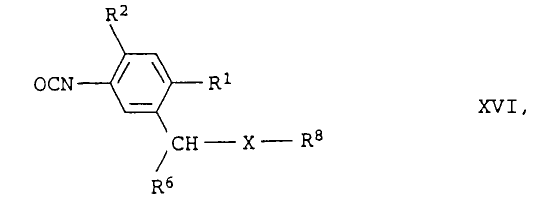 dans laquelle les symboles R¹, R² et R⁶ à R⁹ ont les significations indiquées dans la revendication 1.

14. Dérivés de l'aniline répondant à la formule XVII 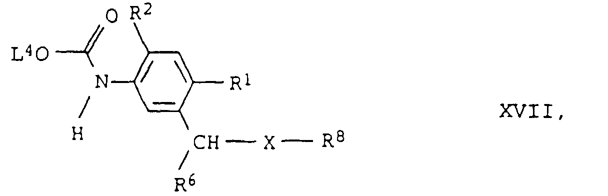 dans laquelle L⁴ représente un groupe alkyle en C1-C4 ou phényle et les symboles R¹, R² et R⁶ à R⁸ ont les significations indiquées dans la revendication 1.

15. Dérivés de l'aniline répondant à la formule XIX 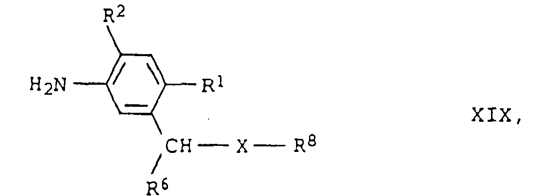 dans laquelle les symboles R¹, R² et R⁶ à R⁸ ont les significations indiquées dans la revendication 1.

16. Dérivés nitrés de formule XXIII 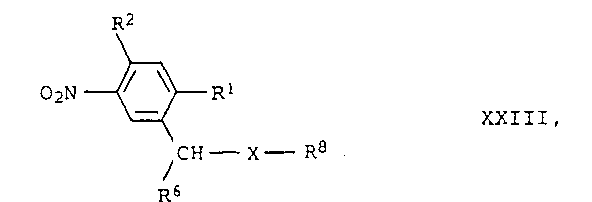 dans laquelle les symboles R¹, R² et R⁶ à R⁸ ont les significations indiquées dans la revendication 1.
